(19) Europäisches Patentamt
European Patent Office
Office européen des brevets

(11) Publication number: 0 535 231 B1

(12) **EUROPEAN PATENT SPECIFICATION**

(45) Date of publication of patent specification: **03.05.95**

(51) Int. Cl.[6]: **C07D 405/04**, C07H 19/06, A61K 31/70

(21) Application number: **91911192.2**

(22) Date of filing: **13.06.91**

(86) International application number: **PCT/JP91/00797**

(87) International publication number: **WO 91/19713 (26.12.91 91/29)**

The file contains technical information submitted after the application was filed and not included in this specification

(54) **PYRIMIDINE NUCLEOSIDE DERIVATIVE.**

(30) Priority: **15.06.90 JP 156688/90**

(43) Date of publication of application: **07.04.93 Bulletin 93/14**

(45) Publication of the grant of the patent: **03.05.95 Bulletin 95/18**

(84) Designated Contracting States: **AT BE CH DE DK ES FR GB GR IT LI LU NL SE**

(56) References cited:
**ES-A- 2 007 094**

**NUCLEIC ACIDS SYMPOSIUM SERIES, no. 22, 1990, pages 51-52; A. MATSUDA et al.: "Synthesis of a new potent antitumour nucleoside, 2'-C-cyano-2'-deoxy-1-B-D-arabinofuranosycytosine"**

(73) Proprietor: **SANKYO COMPANY LIMITED**
**5-1 Nihonbashi Honcho 3-chome**
**Chuo-ku,**
**Tokyo 103 (JP)**

(72) Inventor: **MATSUDA, Akira, 10-501,**
**Minamishinkawa**
**Koumuinshukusha,**
**Nishi 13-chome,**
**Kita 23Joh**
**Kita-ku,**
**Sapporo-shi,**
**Hokkaido 060 (JP)**
Inventor: **SASAKI, Takuma**
**Heiwa-Shukusha (C)-57-11,**
**18-15, Hewamachi 3-chome**
**Kanazawa-shi,**
**Ishikawa-ken 921 (JP)**
Inventor: **UEDA, Tohru**

**deceased (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

**CHEMICAL ABSTRACTS, vol. 110, no. 25, June 19, 1989, Columbus, Ohio, USA; D. HAEBICH: "Synthesis of 3'-cyano-3'-deoxy-beta-D-arabino-nucleosides", abstract-no. 232006z, Synthesis, (12), 943-7**

**CHEMICAL ABSTRACTS, vol. 111, no. 5, July 31, 1989, Columbus, Ohio, USA; Y. DONG: "Synthesis of 3'-cyano-2',3'-dideoxyadenosine and 2',3'-dideoxy-3'-formyladenosine", abstract-no. 39809f, J. Org. Chem., 54(13), 3240-2**

(74) Representative: **Gibson, Christian John Robert et al**
**MARKS & CLERK,**
**57/60 Lincoln's Inn Fields**
**London WC2A 3LS (GB)**

## Description

The present invention relates to novel pyrimidine nucleoside derivatives having excellent antitumour activity.

Various commercially available antitumour agents are known which are metabolism antagonists, and which belong to the pyrimidine series, such as: 5-fluorouracil [Duschinsky, R., et al., J. Am. Chem. Soc., 79, 4559 (1957)]; Tegafur [Hiller, SA., et al., Dokl. Akad. Nauk USSR, 176, 332 (1967)]; UFT [Fujii, S., et al., Gann, 69, 763 (1978)]; Carmofur [Hoshi, A., et al., Gann, 67, 725 (1976)]; Doxyfluridine [Cook, A. F., et al., J. Med. Chem., 22, 1330 (1979)]; Cytarabine [Evance, J. S., et al., Proc. Soc. Exp. Bio. Med., 106, 350 (1961)-]; Ancytabine [Hoshi, A., et al., Gann, 63, 353, (1972)]; and Enocytabine [Aoshima, M., et al., Cancer Res., 36, 2726 (1976)].

Known pyrimidine mononucleosides having a cyano group on the ribose moiety are restricted to 3'-cyanothymine nucleoside and 3'-cyanouracil nucleoside derivatives (Japanese Unexamined Patent Publication Nos. Hei-2-83392, Hei-2-104586 and Hei-2-503002).

The present invention relates to novel antimetabolites which have superior activity to those described above. In the compounds of the present invention, a cyano group is introduced in the 2'-position of the sugar moiety of a pyrimidine nucleosides, such compounds having strong antitumour activity in various tumour systems and also providing intermediates for producing other such compounds.

Thus, in a first aspect, the present invention provides compounds having the general formula:

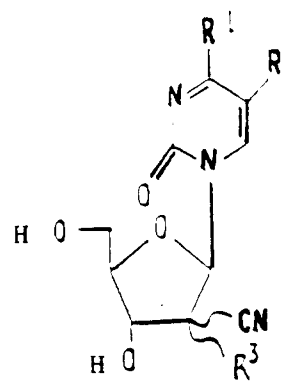

or the general formula:

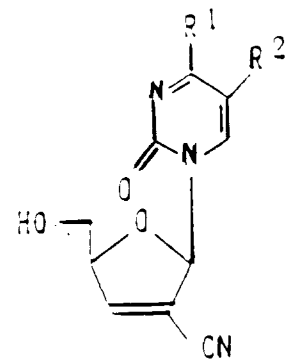

[wherein $R^1$ represents a hydroxyl group or an amino group optionally having a substituent selected from the group consisting of substituents (a) and (b) below;
$R^2$ represents a hydrogen atom or an alkyl group having from 1 to 4 carbon atoms;
$R^3$ represents a hydrogen atom or a hydroxyl group; and
substituents (a)
aliphatic acyl groups having from 1 to 4 carbon atoms, and aromatic acyl groups having from 7 to 11 carbon atoms, the ring having an optional substituent thereon selected from benzoyl, $\alpha$-naphthoyl and $\beta$-naphthoyl groups;
substituents (b)

alkoxycarbonyl groups wherein the alkyl part has from 1 to 4 carbon atoms, alkenyloxycarbonyl wherein the alkenyl part has from 2 to 4 carbon atoms, aralkyloxycarbonyl groups having from 8 to 12 carbon atoms, the ring having an optional substituent thereon selected from alkyl groups having from 1 to 4 carbon atoms, alkoxy groups having from 1 to 4 carbon atoms and aliphatic acyloxy groups having from 1 to 4 carbon atoms];

and pharmacologically acceptable non-toxic salts thereof.

The present invention also provides methods for the manufacture of the above compounds (given in more detail below), as well as the use of the compounds in therapy, and the use of the compounds for the manufacture of a medicament for the treatment or prophylaxis of tumours.

Compounds of formulae (1) and (2) exhibit strong antitumour activities to P388 cell transplanted into mice, as well as to various human cancers. These compounds can easily be absorbed after oral administration, and possess low toxicity with only mild side effects.

Accordingly, the compounds of formulae (1) and (2) are useful, as novel pyrimidine nucleoside type antitumour agents, for the treatment and prophylaxis of tumorigenic diseases.

The present invention furthermore provides compounds of formula (I'):

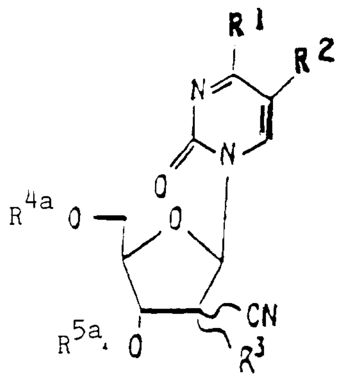

(I')

in which $R^1$, $R^2$ and $R^3$ are as defined above and $R^{4a}$ and $R^{5a}$ together form a group $-R^6R^7Si-O-SiR^{6'}R^{7'}-$, wherein $R^6$, $R^{6'}$, $R^7$ and $R^{7'}$ are the same or different and each represents an alkyl group having from 1 to 4 carbon atoms. These compounds are useful as intermediates for the production of antitumour agents.

In the compounds of the present invention, when $R^1$ is substituted by an aliphatic acyl group having 1 to 4 carbon atoms, such substituent may be selected from formyl, acetyl, propionyl, butyryl and isopropionyl, and is preferably an aliphatic acyl having 1 or 2 carbon atoms.

Where $R^1$ is substituted by an aromatic acyl group having 7 to 11 carbon atoms, such substituent may be selected from benzoyl, $\alpha$-naphthoyl and $\beta$-naphthoyl, and is preferably benzoyl.

Where $R^1$ is substituted by a substituent (a) which is an aromatic acyl group which is itself substituted on the ring, then the ring substituent may be selected from an alkyl group having from 1 to 4 carbon atoms, an alkoxy group having from 1 to 4 carbon atoms and an aliphatic acyl group having from 1 to 4 carbon atoms, and is preferably selected from methyl, ethyl, methoxy, ethoxy and acetyl groups.

The $C_1$ - $C_4$ alkyl moiety of alkoxycarbonyl groups described herein may be selected from methyl, ethyl, n-propyl, isopropyl, n-butyl, isobutyl and t-butyl, and is preferably selected from methyl and t-butyl.

In compounds of the present invention, where a substituent (b) is present and represents an alkenyloxycarbonyl group, then the alkenyl moiety may be selected from vinyl, allyl, isopropenyl, 1-butenyl and 2-butenyl, and is preferably allyl.

Where substituent (b) is present and represents an aralkyloxycarbonyl group having 8 to 12 carbon atoms, then the aralkyl moiety may be selected from benzyl, phenethyl, $\alpha$-naphthylmethyl and $\beta$-naphthylethyl, and is preferably benzyl.

Where substituent (b) is present and represents an aralkyloxycarbonyl group having a substituent on the ring, the substituent may be selected from alkyl groups having from 1 to 4 carbon atoms, alkoxy groups having from 1 to 4 carbon atoms and aliphatic acyloxy groups having from 1 to 4 carbon atoms, and are preferably selected from methyl, ethyl, methoxy, ethoxy and acetoxy.

Preferred meanings for $R^1$ are: hydroxyl, amino, amino substituted with a $C_1$ - $C_2$ aliphatic acyl, amino substituted with an aromatic acyl having 7 carbon atoms and which is optionally substituted on the ring, amino substituted with a $C_1$ - $C_4$ alkoxycarbonyl group, amino substituted with an alkenyloxycarbonyl having a $C_3$ alkenyl moiety, amino substituted with a $C_8$ aralkyloxycarbonyl group which is optionally

substituted on the ring, and is more preferably selected from a hydroxyl group, an amino group, an amino group substituted with an aliphatic acyl having 1 or 2 carbon atoms, and an amino group substituted with an aromatic acyl having 7 carbon atoms, and is most preferably a hydroxyl group or an amino group.

Where $R^2$ represents an alkyl group having 1 to 4 carbon atoms, this may be selected from methyl, ethyl, propyl, isopropyl, butyl, isobutyl and t-butyl groups, and is preferably a methyl group. Particularly preferred meanings for $R^2$ are hydrogen and methyl.

The groups $R^6$, $R^{6'}$, $R^7$ or $R^{7'}$ may be selected from methyl, ethyl, propyl, isopropyl, butyl, isobutyl and t-butyl groups, and preferably represent an isopropyl group.

$R^4$ and $R^5$ may each represent a hydrogen atom or together may represent, for example, a tetramethyldisiloxdiyl group, a tetraethyldisiloxdiyl group, a tetrapropyldisiloxdiyl group, a tetraisopropyldisiloxdiyl group, a tetrabutyldisiloxdiyl group, a diethyldiisopropyldisiloxdiyl group or a dibutyldiisopropyldisiloxdiyl group, and preferably each represent a hydrogen atom or together represent a tetraisopropyldisiloxdiyl group, most preferably each representing a hydrogen atom.

Pharmacologically acceptable nontoxic salts of compounds having the above general formulae (1) or (2) of the present invention include salts of mineral acids such as hydrochloride, hydrobromide and sulphate, organic sulphonates such as methanesulphonate and benzenesulphonate, aliphatic carboxylates such as acetate, propionate, butyrate and caproate, and aromatic carboxylates such as benzoate.

Preferred salts are those of mineral acids, particularly hydrochloric acid, and aliphatic carboxylates, particularly acetic acid.

Preferred classes of compound within the definitions of compounds of formulae (1) and (2) are as follows:

Compounds wherein $R^1$ represents a hydroxyl group or an amino group and optionally has a substituent selected from the group consisting of substituents (a') and (b') below; and $R^4$ and $R^5$ each represent a hydrogen atom or together represent a tetraisopropyldisiloxdiyl group;

substituents (a')

aliphatic acyl groups having from 1 to 2 carbon atoms, and aromatic acyl groups having 7 carbon atoms, the ring having an optional substituent thereon;

substituents (b')

alkoxycarbonyl groups wherein the alkyl part has from 1 to 4 carbon atoms, alkenyloxycarbonyl groups wherein the alkenyl part has 3 carbon atoms, aralkyloxycarbonyl groups having 8 or 9 carbon atoms and which optionally have a substituent on the ring;

Compounds wherein $R^1$ represents a hydroxyl group or an amino group and optionally has a substituent (a') as defined; $R^2$ represents a hydrogen atom or a methyl group; and $R^4$ and $R^5$ each represent a hydrogen atom;

and

Compounds wherein $R^1$ represents a hydroxyl group or an amino group; $R^2$ represents a hydrogen atom or a methyl group; and $R^4$ and $R^5$ each represent a hydrogen atom.

The following Tables 1, 2 and 3 exemplify compounds having the general formulae (1) and (2), but do not necessarily provide a definitive list.

Table 1, Table 2 and Table 3 show compounds having the general formula A, general formula B and general formula C, respectively. In the Tables, the abbreviations have the following meanings:

| | |
|---|---|
| Et | = Ethyl |
| Pr | = Propyl |
| tBu | = t-Butyl |
| AL | = Allyl |
| Ac | = Acetyl |
| Bz | = Benzoyl |
| BzpMe | = p-methylbenzoyl |
| BzpOMe | = p-methoxybenzoyl |
| By | = Benzyl |
| BypOAc | = p-acetoxybenzyl |

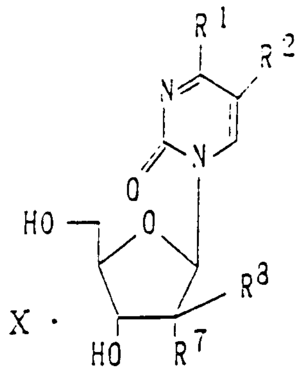

(A)

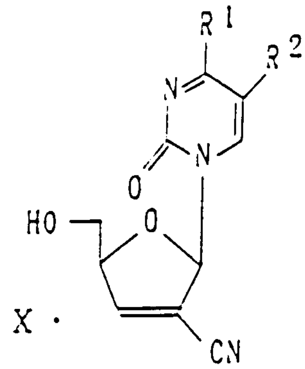

(B)

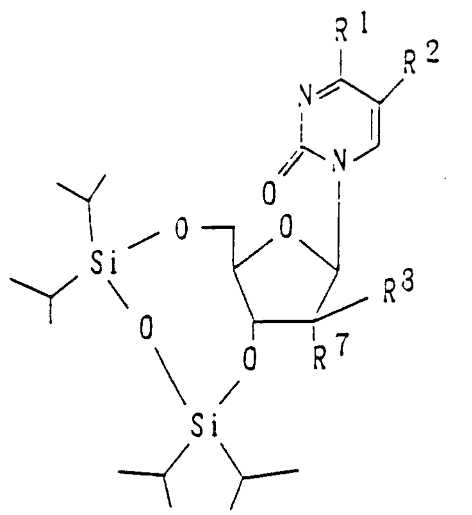

(C)

Table 1

| No. | $R^1$ | $R^2$ | $R^7$ | $R^8$ | X |
|---|---|---|---|---|---|
| 1-1 | $NH_2$ | H | H | CN | - |
| 1-2 | $NH_2$ | $CH_3$ | H | CN | - |
| 1-3 | $NH_2$ | $CH_3CH_2$ | H | CN | - |
| 1-4 | $NH_2$ | $CH_3(CH_2)_2$ | H | CN | - |
| 1-5 | $NH_2$ | $(CH_3)_2CH$ | H | CN | - |
| 1-6 | $NH_2$ | $CH_3(CH_2)_3$ | H | CN | - |
| 1-7 | $NH_2$ | H | H | CN | HCl |
| 1-8 | $NH_2$ | $CH_3$ | H | CN | HCl |
| 1-9 | $NH_2$ | $CH_3CH_2$ | H | CN | HCl |
| 1-10 | $NH_2$ | $CH_3(CH_2)_2$ | H | CN | HCl |
| 1-11 | $NH_2$ | $(CH_3)_2CH$ | H | CN | HCl |
| 1-12 | $NH_2$ | $CH_3(CH_2)_3$ | H | CN | HCl |
| 1-13 | OH | H | H | CN | - |
| 1-14 | OH | $CH_3$ | H | CN | - |
| 1-15 | OH | $CH_3CH_2$ | H | CN | - |
| 1-16 | OH | $CH_3(CH_2)_2$ | H | CN | - |
| 1-17 | OH | $(CH_3)_2CH$ | H | CN | - |
| 1-18 | OH | $CH_3(CH_2)_3$ | H | CN | - |
| 1-19 | $NH_2$ | H | CN | OH | - |
| 1-20 | $NH_2$ | $CH_3$ | CN | OH | - |
| 1-21 | $NH_2$ | $CH_3CH_2$ | CN | OH | - |
| 1-22 | $NH_2$ | $CH_3(CH_2)_2$ | CN | OH | - |
| 1-23 | $NH_2$ | $(CH_3)_2CH$ | CN | OH | - |
| 1-24 | $NH_2$ | $CH_3(CH_2)_3$ | CN | OH | - |
| 1-25 | $NH_2$ | H | CN | OH | HCl |
| 1-26 | $NH_2$ | $CH_3$ | CN | OH | HCl |
| 1-27 | $NH_2$ | $CH_3CH_2$ | CN | OH | HCl |
| 1-28 | $NH_2$ | $CH_3(CH_2)_2$ | CN | OH | HCl |
| 1-29 | $NH_2$ | $(CH_3)_2CH$ | CN | OH | HCl |
| 1-30 | $NH_2$ | $CH_3(CH_2)_3$ | CN | OH | HCl |
| 1-31 | OH | H | CN | OH | - |
| 1-32 | OH | $CH_3$ | CN | OH | - |
| 1-33 | OH | $CH_3CH_2$ | CN | OH | - |

| | | | | | |
|---|---|---|---|---|---|
| 1-34 | OH | $CH_3(CH_2)_2$ | CN | OH | - |
| 1-35 | OH | $(CH_3)_2CH$ | CN | OH | - |
| 1-36 | OH | $CH_3(CH_2)_3$ | CN | OH | - |
| 1-37 | $NH_2$ | H | CN | H | - |
| 1-38 | $NH_2$ | $CH_3$ | CN | H | - |
| 1-39 | $NH_2$ | $CH_3CH_2$ | CN | H | - |
| 1-40 | $NH_2$ | $CH_3(CH_2)_2$ | CN | H | - |
| 1-41 | $NH_2$ | $(CH_3)_2CH$ | CN | H | - |
| 1-42 | $NH_2$ | $CH_3(CH_2)_3$ | CN | H | - |
| 1-43 | $NH_2$ | H | CN | H | HCl |
| 1-44 | $NH_2$ | $CH_3$ | CN | H | HCl |
| 1-45 | $NH_2$ | $CH_3CH_2$ | CN | H | HCl |
| 1-46 | $NH_2$ | $CH_3(CH_2)_2$ | CN | H | HCl |
| 1-47 | $NH_2$ | $(CH_3)_2CH$ | CN | H | HCl |
| 1-48 | $NH_2$ | $CH_3(CH_2)_3$ | CN | H | HCl |
| 1-49 | OH | H | CN | H | - |
| 1-50 | OH | $CH_3$ | CN | H | - |
| 1-51 | OH | $CH_3CH_2$ | CN | H | - |
| 1-52 | OH | $CH_3(CH_2)_2$ | CN | H | - |
| 1-53 | OH | $(CH_3)_2CH$ | CN | H | - |
| 1-54 | OH | $CH_3(CH_2)_3$ | CN | H | - |
| 1-55 | $NH_2$ | H | OH | CN | - |
| 1-56 | $NH_2$ | $CH_3$ | OH | CN | - |
| 1-57 | $NH_2$ | $CH_3CH_2$ | OH | CN | - |
| 1-58 | $NH_2$ | $CH_3(CH_2)_2$ | OH | CN | - |
| 1-59 | $NH_2$ | $(CH_3)_2CH$ | OH | CN | - |
| 1-60 | $NH_2$ | $CH_3(CH_2)_3$ | OH | CN | - |
| 1-61 | $NH_2$ | H | OH | CN | HCl |
| 1-62 | $NH_2$ | $CH_3$ | OH | CN | HCl |
| 1-63 | $NH_2$ | $CH_3CH_2$ | OH | CN | HCl |
| 1-64 | $NH_2$ | $CH_3(CH_2)_2$ | OH | CN | HCl |
| 1-65 | $NH_2$ | $(CH_3)_2CH$ | OH | CN | HCl |
| 1-66 | $NH_2$ | $CH_3(CH_2)_3$ | OH | CN | HCl |
| 1-67 | OH | H | OH | CN | - |
| 1-68 | OH | $CH_3$ | OH | CN | - |
| 1-69 | OH | $CH_3CH_2$ | OH | CN | - |
| 1-70 | OH | $CH_3(CH_2)_2$ | OH | CN | - |

| | | | | | |
|---|---|---|---|---|---|
| 1-71 | OH | $(CH_3)_2CH$ | OH | CN | - |
| 1-72 | OH | $CH_3(CH_2)_3$ | OH | CN | - |
| 1-73 | NHBz | H | H | CN | - |
| 1-74 | NHCOOtBu | H | H | CN | - |
| 1-75 | NHCOOBy | H | H | CN | - |
| 1-76 | NHCOOAL | H | H | CN | - |
| 1-77 | NHBz | $CH_3$ | H | CN | - |
| 1-78 | NHBz | $CH_3CH_2$ | H | CN | - |
| 1-79 | NHBz | $CH_3(CH_2)_2$ | H | CN | - |
| 1-80 | NHBz | $(CH_3)_2CH$ | H | CN | - |
| 1-81 | NHBz | $CH_3(CH_2)_3$ | H | CN | - |
| 1-82 | NHBz | H | CN | H | - |
| 1-83 | NHBz | $CH_3$ | CN | H | - |
| 1-84 | NHBz | $CH_3CH_2$ | CN | H | - |
| 1-85 | NHBz | $CH_3(CH_2)_2$ | CN | H | - |
| 1-86 | NHBz | $(CH_3)_2CH$ | CN | H | - |
| 1-87 | NHBz | $CH_3(CH_2)_3$ | CN | H | - |
| 1-88 | NHAc | H | H | CN | - |
| 1-89 | NHCOH | H | H | CN | - |
| 1-90 | NHCOEt | H | H | CN | - |
| 1-91 | NHCOPr | H | H | CN | - |
| 1-92 | NHAc | $CH_3$ | H | CN | - |
| 1-93 | NHAc | $CH_3CH_2$ | H | CN | - |
| 1-94 | NHAc | $CH_3(CH_2)_2$ | H | CN | - |
| 1-95 | NHAc | $(CH_3)_2CH$ | H | CN | - |
| 1-96 | NHAc | $CH_3(CH_2)_3$ | H | CN | - |
| 1-97 | NHAc | H | OH | CN | - |
| 1-98 | NHAc | $CH_3$ | OH | CN | - |
| 1-99 | NHAc | $CH_3CH_2$ | OH | CN | - |
| 1-100 | NHAc | $CH_3(CH_2)_2$ | OH | CN | - |
| 1-101 | NHAc | $(CH_3)_2CH$ | OH | CN | - |
| 1-102 | NHAc | $CH_3(CH_2)_3$ | OH | CN | - |
| 1-103 | NHAc | H | CN | H | - |
| 1-104 | NHAc | $CH_3$ | CN | H | - |
| 1-105 | NHAc | $CH_3CH_2$ | CN | H | - |
| 1-106 | NHAc | $CH_3(CH_2)_2$ | CN | H | - |
| 1-107 | NHAc | $(CH_3)_2CH$ | CN | H | - |
| 1-108 | NHAc | $CH_3(CH_2)_3$ | CN | H | - |

Table 2

| No. | $R^1$ | $R^2$ | X |
|---|---|---|---|
| 2-1 | $NH_2$ | H | - |
| 2-2 | $NH_2$ | $CH_3$ | - |
| 2-3 | $NH_2$ | $CH_3CH_2$ | - |
| 2-4 | $NH_2$ | $CH_3(CH_2)_2$ | - |
| 2-5 | $NH_2$ | $(CH_3)_2CH$ | - |
| 2-6 | $NH_2$ | $CH_3(CH_2)_3$ | - |
| 2-7 | $NH_2$ | H | HCl |
| 2-8 | $NH_2$ | $CH_3$ | HCl |
| 2-9 | $NH_2$ | $CH_3CH_2$ | HCl |
| 2-10 | $NH_2$ | $CH_3(CH_2)_2$ | HCl |
| 2-11 | $NH_2$ | $(CH_3)_2CH$ | HCl |
| 2-12 | $NH_2$ | $CH_3(CH_2)_3$ | HCl |
| 2-13 | OH | H | - |
| 2-14 | OH | $CH_3$ | - |
| 2-15 | OH | $CH_3CH_2$ | - |
| 2-16 | OH | $CH_3(CH_2)_2$ | - |
| 2-17 | OH | $(CH_3)_2CH$ | - |
| 2-18 | OH | $CH_3(CH_2)_3$ | - |
| 2-19 | NHAc | H | - |
| 2-20 | NHAc | $CH_3$ | - |
| 2-21 | NHAc | $CH_3CH_2$ | - |
| 2-22 | NHAc | $CH_3(CH_2)_2$ | - |
| 2-23 | NHAc | $(CH_3)_2CH$ | - |
| 2-24 | NHAc | $CH_3(CH_2)_3$ | - |
| 2-25 | NHBzpMe | H | - |
| 2-26 | NHBzpOMe | $CH_3$ | - |
| 2-27 | NHCOOtBu | $CH_3CH_2$ | - |
| 2-28 | NHCOOBy | $CH_3(CH_2)_2$ | - |
| 2-29 | NHCOOAL | $(CH_3)_2CH$ | - |
| 2-30 | NHCOOBypOAc | $CH_3(CH_2)_3$ | - |
| 2-31 | NHBz | H | - |

| | | | |
|---|---|---|---|
| 2-32 | NHBz | $CH_3$ | - |
| 2-33 | NHBz | $CH_3CH_2$ | - |
| 2-34 | NHBz | $CH_3(CH_2)_2$ | - |
| 2-35 | NHBz | $(CH_3)_2CH$ | - |
| 2-36 | NHBz | $CH_3(CH_2)_3$ | - |
| 2-37 | NHAc | H | - |
| 2-38 | NHAc | $CH_3$ | - |
| 2-39 | NHAc | $CH_3CH_2$ | - |
| 2-40 | NHAc | $CH_3(CH_2)_2$ | - |
| 2-41 | NHAc | $(CH_3)_2CH$ | - |
| 2-42 | NHAc | $CH_3(CH_2)_3$ | - |

Table 3

| No. | $R^1$ | $R^2$ | $R^7$ | $R^8$ | X |
|---|---|---|---|---|---|
| 3-1 | $NH_2$ | H | H | CN | - |
| 3-2 | $NH_2$ | $CH_3$ | H | CN | - |
| 3-3 | $NH_2$ | $CH_3CH_2$ | H | CN | - |
| 3-4 | $NH_2$ | $CH_3(CH_2)_2$ | H | CN | - |
| 3-5 | $NH_2$ | $(CH_3)_2CH$ | H | CN | - |
| 3-6 | $NH_2$ | $CH_3(CH_2)_3$ | H | CN | - |
| 3-7 | OH | H | H | CN | - |
| 3-8 | OH | $CH_3$ | H | CN | - |
| 3-9 | OH | $CH_3CH_2$ | H | CN | - |
| 3-10 | OH | $CH_3(CH_2)_2$ | H | CN | - |
| 3-11 | OH | $(CH_3)_2CH$ | H | CN | - |
| 3-12 | OH | $CH_3(CH_2)_3$ | H | CN | - |
| 3-13 | $NH_2$ | H | CN | OH | - |
| 3-14 | $NH_2$ | $CH_3$ | CN | OH | - |
| 3-15 | $NH_2$ | $CH_3CH_2$ | CN | OH | - |
| 3-16 | $NH_2$ | $CH_3(CH_2)_2$ | CN | OH | - |

| | | | | | |
|---|---|---|---|---|---|
| 3-17 | $NH_2$ | $(CH_3)_2CH$ | CN | OH | - |
| 3-18 | $NH_2$ | $CH_3(CH_2)_3$ | CN | OH | - |
| 3-19 | OH | H | CN | OH | - |
| 3-20 | OH | $CH_3$ | CN | OH | - |
| 3-21 | OH | $CH_3CH_2$ | CN | OH | - |
| 3-22 | OH | $CH_3(CH_2)_2$ | CN | OH | - |
| 3-23 | OH | $(CH_3)_2CH$ | CN | OH | - |
| 3-24 | OH | $CH_3(CH_2)_3$ | CN | OH | - |
| 3-25 | $NH_2$ | H | CN | H | - |
| 3-26 | $NH_2$ | $CH_3$ | CN | H | - |
| 3-27 | $NH_2$ | $CH_3CH_2$ | CN | H | - |
| 3-28 | $NH_2$ | $CH_3(CH_2)_2$ | CN | H | - |
| 3-29 | $NH_2$ | $(CH_3)_2CH$ | CN | H | - |
| 3-30 | $NH_2$ | $CH_3(CH_2)_3$ | CN | H | - |
| 3-31 | OH | H | CN | H | - |
| 3-32 | OH | $CH_3$ | CN | H | - |
| 3-33 | OH | $CH_3CH_2$ | CN | H | - |
| 3-34 | OH | $CH_3(CH_2)_2$ | CN | H | - |
| 3-35 | OH | $(CH_3)_2CH$ | CN | H | - |
| 3-36 | OH | $CH_3(CH_2)_3$ | CN | H | - |
| 3-37 | $NH_2$ | H | OH | CN | - |
| 3-38 | $NH_2$ | $CH_3$ | OH | CN | - |
| 3-39 | $NH_2$ | $CH_3CH_2$ | OH | CN | - |
| 3-40 | $NH_2$ | $CH_3(CH_2)_2$ | OH | CN | - |
| 3-41 | $NH_2$ | $(CH_3)_2CH$ | OH | CN | - |
| 3-42 | $NH_2$ | $CH_3(CH_2)_3$ | OH | CN | - |
| 3-43 | OH | H | OH | CN | - |
| 3-44 | OH | $CH_3$ | OH | CN | - |
| 3-45 | OH | $CH_3CH_2$ | OH | CN | - |
| 3-46 | OH | $CH_3(CH_2)_2$ | OH | CN | - |
| 3-47 | OH | $(CH_3)_2CH$ | OH | CN | - |
| 3-48 | OH | $CH_3(CH_2)_3$ | OH | CN | - |
| 3-49 | NHAc | H | H | CN | - |
| 3-50 | NHAc | $CH_3$ | H | CN | - |
| 3-51 | NHAc | $CH_3CH_2$ | H | CN | - |
| 3-52 | NHAc | $CH_3(CH_2)_2$ | H | CN | - |
| 3-53 | NHAc | $(CH_3)_2CH$ | H | CN | - |

| | | | | | |
|---|---|---|---|---|---|
| 3-54 | NHAc | $CH_3(CH_2)_3$ | H | CN | - |
| 3-55 | NHAc | H | CN | OH | - |
| 3-56 | NHAc | $CH_3$ | CN | OH | - |
| 3-57 | NHAc | $CH_3CH_2$ | CN | OH | - |
| 3-58 | NHAc | $CH_3(CH_2)_2$ | CN | OH | - |
| 3-59 | NHAc | $(CH_3)_2CH$ | CN | OH | - |
| 3-60 | NHAc | $CH_3(CH_2)_3$ | CN | OH | - |
| 3-61 | NHAc | H | CN | H | - |
| 3-62 | NHAc | $CH_3$ | CN | H | - |
| 3-63 | NHAc | $CH_3CH_2$ | CN | H | - |
| 3-64 | NHAc | $CH_3(CH_2)_2$ | CN | H | - |
| 3-65 | NHAc | $(CH_3)_2CH$ | CN | H | - |
| 3-66 | NHAc | $CH_3(CH_2)_3$ | CN | H | - |
| 3-67 | NHAc | H | OH | CN | - |
| 3-68 | NHAc | $CH_3$ | OH | CN | - |
| 3-69 | NHAc | $CH_3CH_2$ | OH | CN | - |
| 3-70 | NHAc | $CH_3(CH_2)_2$ | OH | CN | - |
| 3-71 | NHAc | $(CH_3)_2CH$ | OH | CN | - |
| 3-72 | NHAc | $CH_3(CH_2)_3$ | OH | CN | - |
| 3-73 | NHBz | H | H | CN | - |
| 3-74 | NHBz | $CH_3$ | H | CN | - |
| 3-75 | NHBz | $CH_3CH_2$ | H | CN | - |
| 3-76 | NHBz | $CH_3(CH_2)_2$ | H | CN | - |
| 3-77 | NHBz | $(CH_3)_2CH$ | H | CN | - |
| 3-78 | NHBz | $CH_3(CH_2)_3$ | H | CN | - |
| 3-79 | NHBz | H | CN | OH | - |
| 3-80 | NHBz | $CH_3$ | CN | OH | - |
| 3-81 | NHBz | $CH_3CH_2$ | CN | OH | - |
| 3-82 | NHBz | $CH_3(CH_2)_2$ | CN | OH | - |
| 3-83 | NHBz | $(CH_3)_2CH$ | CN | OH | - |
| 3-84 | NHBz | $CH_3(CH_2)_3$ | CN | OH | - |
| 3-85 | NHBz | H | CN | H | - |
| 3-86 | NHBz | $CH_3$ | CN | H | - |
| 3-87 | NHBz | $CH_3CH_2$ | CN | H | - |
| 3-88 | NHBz | $CH_3(CH_2)_2$ | CN | H | - |
| 3-89 | NHBz | $(CH_3)_2CH$ | CN | H | - |
| 3-90 | NHBz | $CH_3(CH_2)_3$ | CN | H | - |

| | | | | | |
|---|---|---|---|---|---|
| 3-91 | NHBz | H | OH | CN | - |
| 3-92 | NHBz | $CH_3$ | OH | CN | - |
| 3-93 | NHBz | $CH_3CH_2$ | OH | CN | - |
| 3-94 | NHBz | $CH_3(CH_2)_2$ | OH | CN | - |
| 3-95 | NHBz | $(CH_3)_2CH$ | OH | CN | - |
| 3-96 | NHBz | $CH_3(CH_2)_3$ | OH | CN | - |

Of the compounds listed above, the following are preferred, that is to say, Compounds No. 1-1, 1-2, 1-7, 1-8, 1-13, 1-14, 1-19, 1-20, 1-25, 1-26, 1-31, 1-32, 1-37, 1-38, 1-43, 1-44, 1-49, 1-50, 1-55, 1-56, 1-61, 1-62, 1-67, 1-68, 1-73, 1-77, 1-82, 1-83, 1-88, 1-89, 1-92, 1-97, 1-98, 1-103, 1-104, 2-1, 2-2, 2-7, 2-8, 2-13, 2-14, 2-19, 2-20, 2-25, 2-26, 2-31, 2-32, 2-37, 2-38, 3-1, 3-2, 3-7, 3-8, 3-13, 3-14, 3-19, 3-20, 3-25, 3-26, 3-31, 3-32, 3-37, 3-38, 3-43, 3-44, 3-49, 3-50, 3-55, 3-56, 3-61, 3-62, 3-67, 3-68, 3-73, 3-74, 3-79, 3-80, 3-85, 3-86, 3-91 and 3-92, and the following are more preferred ones: Compounds No. 1-1, 1-2, 1-7, 1-8, 1-13, 1-14, 1-19, 1-20, 1-25, 1-26, 1-31, 1-32, 1-37, 1-38, 1-43, 1-44, 1-49, 1-50, 1-55, 1-56, 1-61, 1-62, 1-67, 1-68, 1-73, 1-77, 1-82, 1-83, 1-88, 1-89, 1-92, 1-97, 1-98, 1-103, 1-104, 2-1,2-2, 2-7, 2-8, 2-13, 2-14, 2-19, 2-20, 2-25, 2-26, 2-31, 2-32, 2-37 and 2-38.

Still more preferred compounds are Compounds No.: 1-1, 1-2, 1-7, 1-8, 1-13, 1-14, 1-19, 1-20, 1-25, 1-26, 1-31, 1-32, 1-37, 1-38, 1-43, 1-44, 1-49, 1-50, 1-55, 1-56, 1-61, 1-62, 1-67, 1-68, 2-1, 2-2, 2-7, 2-8, 2-13 and 2-14.

The most preferred compounds are Compounds No.: 1-1, 1-7, 1-13, 1-14, 1-19, 1-25, 1-31, 1-32, 1-37, 1-43, 1-49, 1-50, 1-55, 1-61, 1-67, 1-68, 2-1, 2-7, 2-13 and 2-14.

Particularly preferred compounds of the present invention are:

1-(2'-cyano-β-D-arabinofuranosyl)cytosine;
1-(2'-cyano-β-D-arabinofuranosyl)uracil;
1-(2'-cyano-β-D-arabinofuranosyl)thymine;
1-(2'-cyano-β-D-ribofuranosyl)cytosine;
1-(2'-cyano-β-D-ribofuranosyl)uracil;
1-(2'-cyano-β-D-ribofuranosyl)thymine;
1-(2'-cyano-β-D-2'-deoxyarabinofuranosyl)cytosine;
1-(2'-cyano-β-D-2'-deoxyarabinofuranosyl)uracil;
1-(2'-cyano-β-D-2'-deoxyarabinofuranosyl)thymine;
1-(2'-cyano-β-D-2'-deoxyribofuranosyl)cytosine;
1-(2'-cyano-β-D-2'-deoxyribofuranosyl)uracil;
1-(2'-cyano-β-D-2'-deoxyribofuranosyl)thymine;
1-(2'-cyano-β-D-2',3'-didehydro-2',3'-dideoxyribofuranosyl)cytosine;
1-(2'-cyano-β-D-2',3'-didehydro-2',3'-dideoxyribofuranosyl)uracil;
1-(2'-cyano-β-D-2',3'-didehydro-2',3'-dideoxyribofuranosyl)thymine;

and their pharmacologically acceptable salts.

Compounds of the present invention, and their intermediates, can be prepared by the following processes, each of which constitutes a preferred embodiment of the present invention.

A) A process for preparing a compound represented by the general formula:

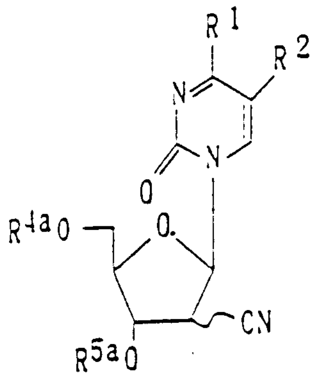

(wherein $R^1$ and $R^2$ are as defined above; and $R^{4a}$ and $R^{5a}$ together form a group of formula $-R^6R^7Si-O-SiR^{6'}R^{7'}-$ [wherein $R^6$, $R^{6'}$, $R^7$ and $R^{7'}$ are as defined above]);
which process comprises reacting a compound represented by the general formula:

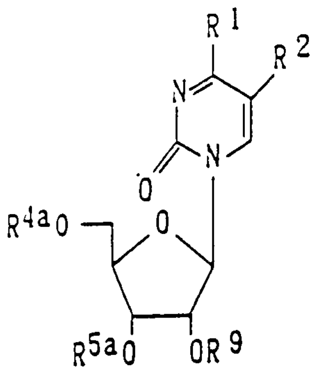

(wherein $R^1$, $R^2$, $R^{4a}$ and $R^{5a}$ are as defined; and $R^9$ represents an alkoxythiocarbonyl group wherein the alkyl part has from 1 to 4 carbon atoms or an arylthiocarbonyl group wherein the aryl pact has from 6 to 10 carbon atoms) with a reducing agent and a cyanolating agent.
B) A process for preparing a compound represented by the general formula:

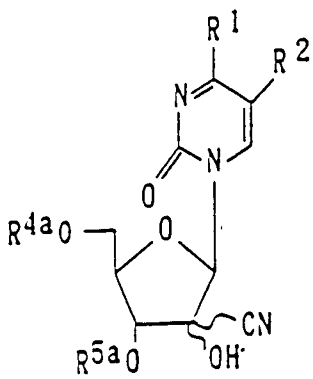

(wherein $R^1$, $R^2$, $R^{4a}$ and $R^{5a}$ are as defined above),
which process comprises reacting a compound represented by the general formula:

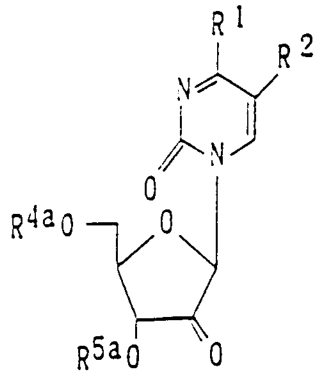

(wherein $R^1$, $R^2$, $R^{4a}$ and $R^{5a}$ ae as defined) with a cyanolating agent.

C) A process for preparing a compound represented by the general formula:

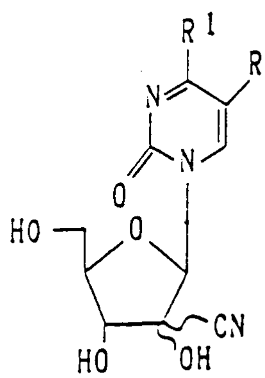

(wherein $R^1$ and $R^2$ are as defined above),

which process comprises reacting a compound represented by the general formula:

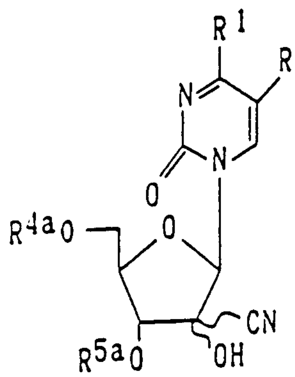

(wherein $R^1$, $R^2$, $R^{4a}$ and $R^{5a}$ are as defined above) with a deprotecting agent.

The above process is particularly preferred when the reactants and conditions are so selected as to produce a compound selected from:

1-(2'-cyano-β-D-arabinofuranosyl)cytosine,
1-(2'-cyano-β-D-arabinofuranosyl)uracil,
1-(2'-cyano-β-D-arabinofuranosyl)thymine,
1-(2'-cyano-β-D-ribofuranosyl)cytosine,
1-(2'-cyano-β-D-ribofuranosyl)uracil, and
1-(2'-cyano-β-D-ribofuranosyl)thymine.

D) A process for preparing a compound represented by the general formula:

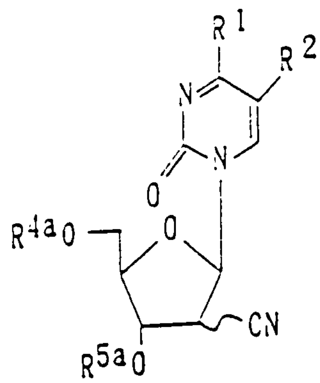

(wherein $R^1$, $R^2$, $R^{4a}$ and $R^{5a}$ are as defined above), which process comprises reacting a compound represented by the general formula:

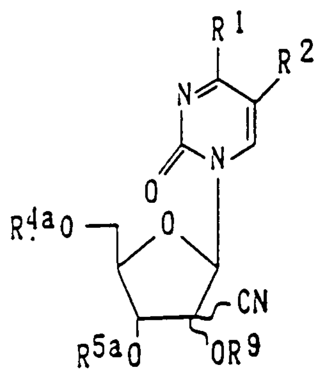

(wherein $R^1$, $R^2$, $R^{4a}$, $R^{5a}$ and $R^9$ are as defined above) with a reducing agent.

E) A process for preparing a compound represented by the general formula:

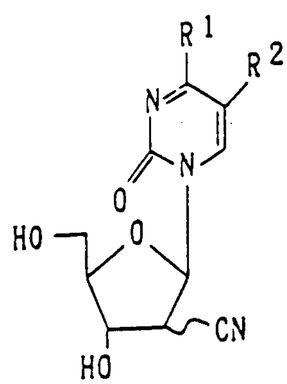

(wherein $R^1$ and $R^2$ are as defined above), which process comprises reacting a compound represented by the general formula:

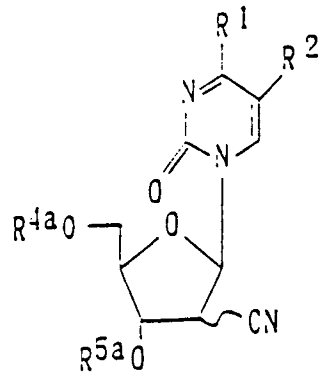

(wherein $R^1$, $R^2$, $R^{4a}$ and $R^{5a}$ are as defined above) with a deprotecting agent.

The above process is particularly preferred when the reactants and conditions are so selected as to produce a compound selected from the group consisting of:
1-(2'-cyano-β-D-2'-deoxy-arabinofuranosyl)cytosine,
1-(2'-cyano-β-D-2'-deoxy-arabinofuranosyl)uracil,
1-(2'-cyano-β-D-2'-deoxy-arabinofuranosyl)thymine,
1-(2'-cyano-β-D-2'-deoxy-ribofuranosyl)cytosine,
1-(2'-cyano-β-D-2'-deoxy-ribofuranosyl)uracil, and
1-(2'-cyano-β-D-2'-deoxy-ribofuranosyl)thymine.

F) A process for preparing a compound represented by the general formula:

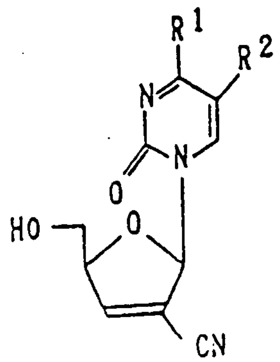

(wherein $R^1$ and $R^2$ are as defined above),
which process comprises reacting a compound represented by the general formula:

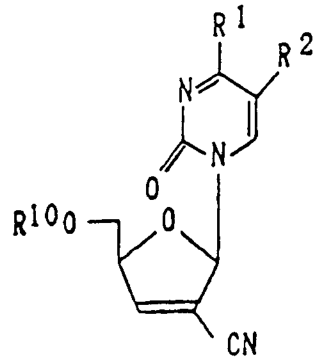

(wherein $R^1$ and $R^2$ are as defined, and $R^{10}$ represents a protective group) with a deprotecting agent.

The above process is particularly preferred when the reactants and conditions are so selected as to produce a compound selected from the group consisting of:
1-(2'-cyano-β-D-2',3'-didehydro-2',3'-dideoxyribofuranosyl)cytosine,
1-(2'-cyano-β-D-2',3'-didehydro-2',3'-dideoxyribofuranosyl)uracil and
1-(2'-cyano-β-D-2',3'-didehydro-2',3'-dideoxyribofuranosyl)thymine.

G) A process for preparing a compound represented by the general formula:

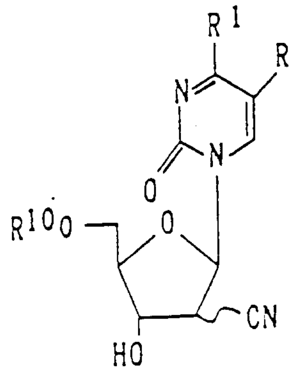

(wherein $R^1$, $R^2$ and $R^{10}$ are as defined above),
which process comprises reacting a compound represented by the general formula:

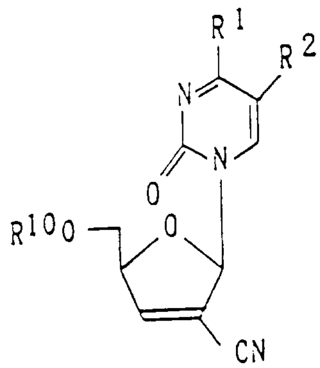

(wherein $R^1$, $R^2$ and $R^{10}$ are as defined) with an alkoxythiocarbonyl halide having a $C_1$ - $C_4$ alkyl moiety, an arylthiocarbonyl halide having a $C_6$ - $C_{10}$ aryl moiety, or thiocarbonyldiimidazole.

The following reaction schemes 1 to 4 are useful in the present invention.

Scheme 1

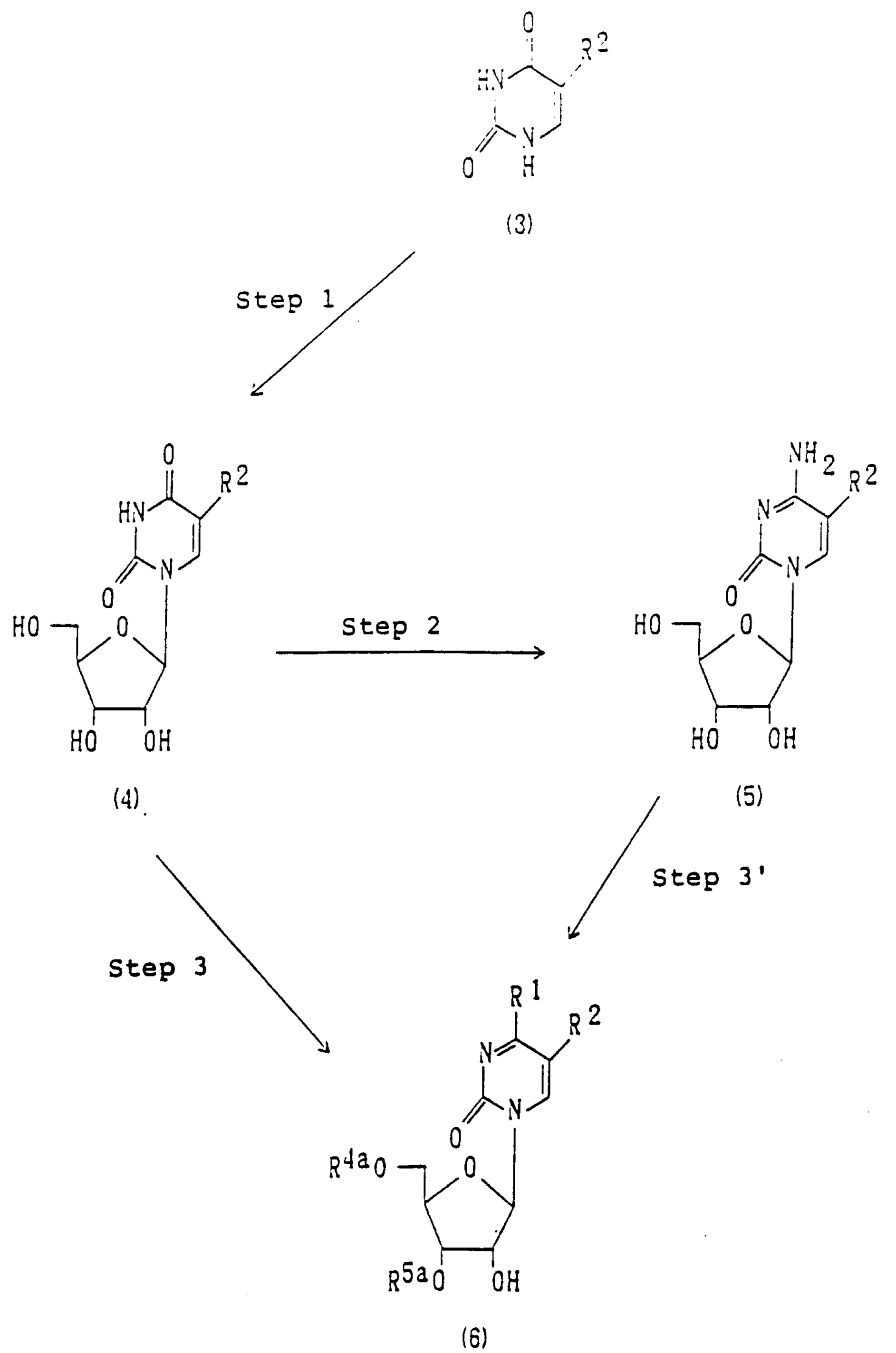


Scheme 1

Scheme 2

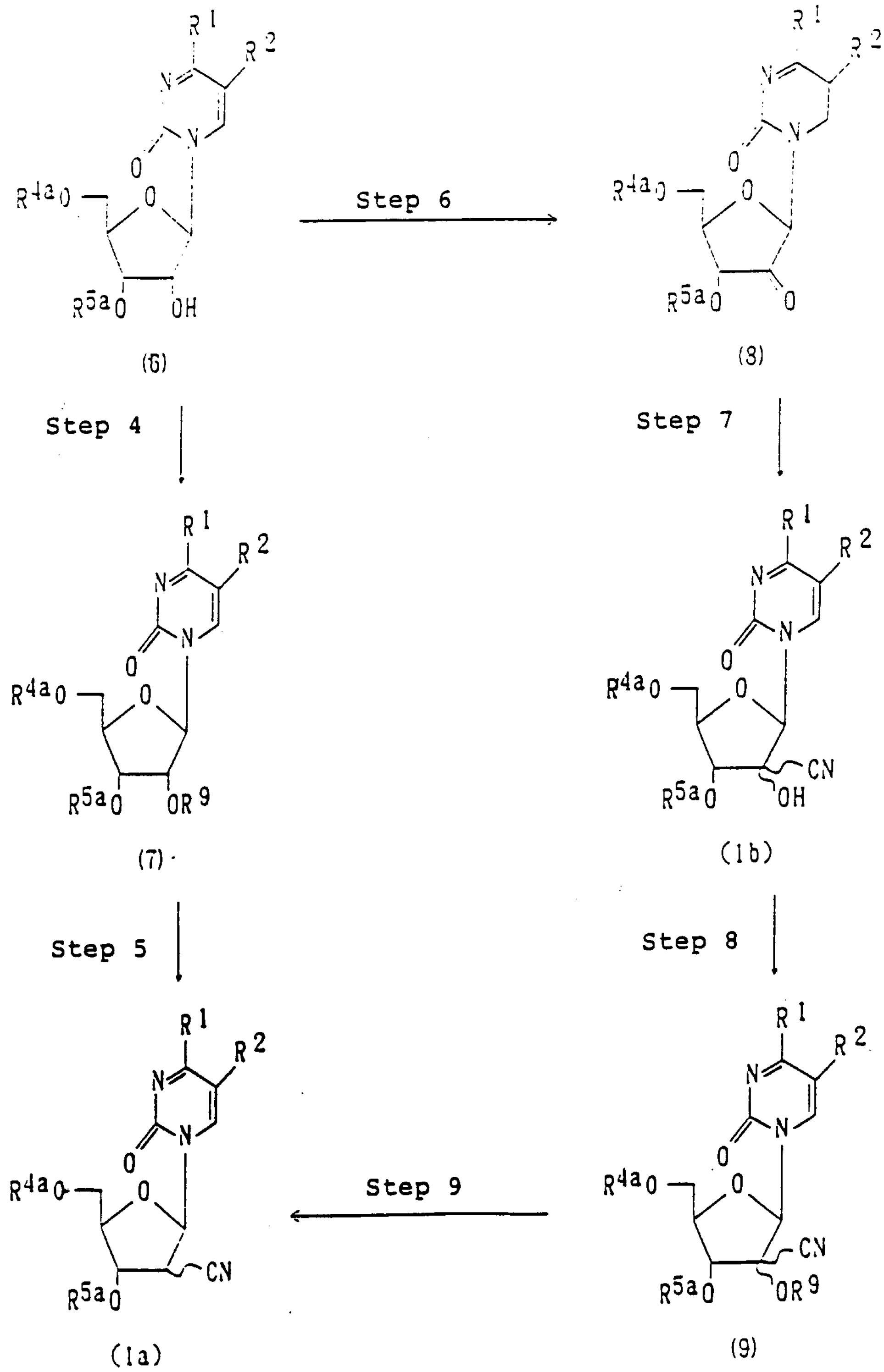


(6) (8) (7) (1b) (1a) (9)

Scheme 3

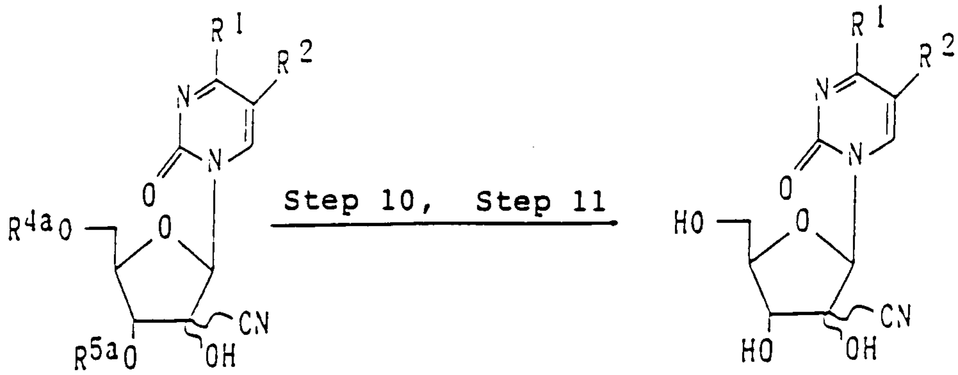


(1b) (1c)

Scheme 4

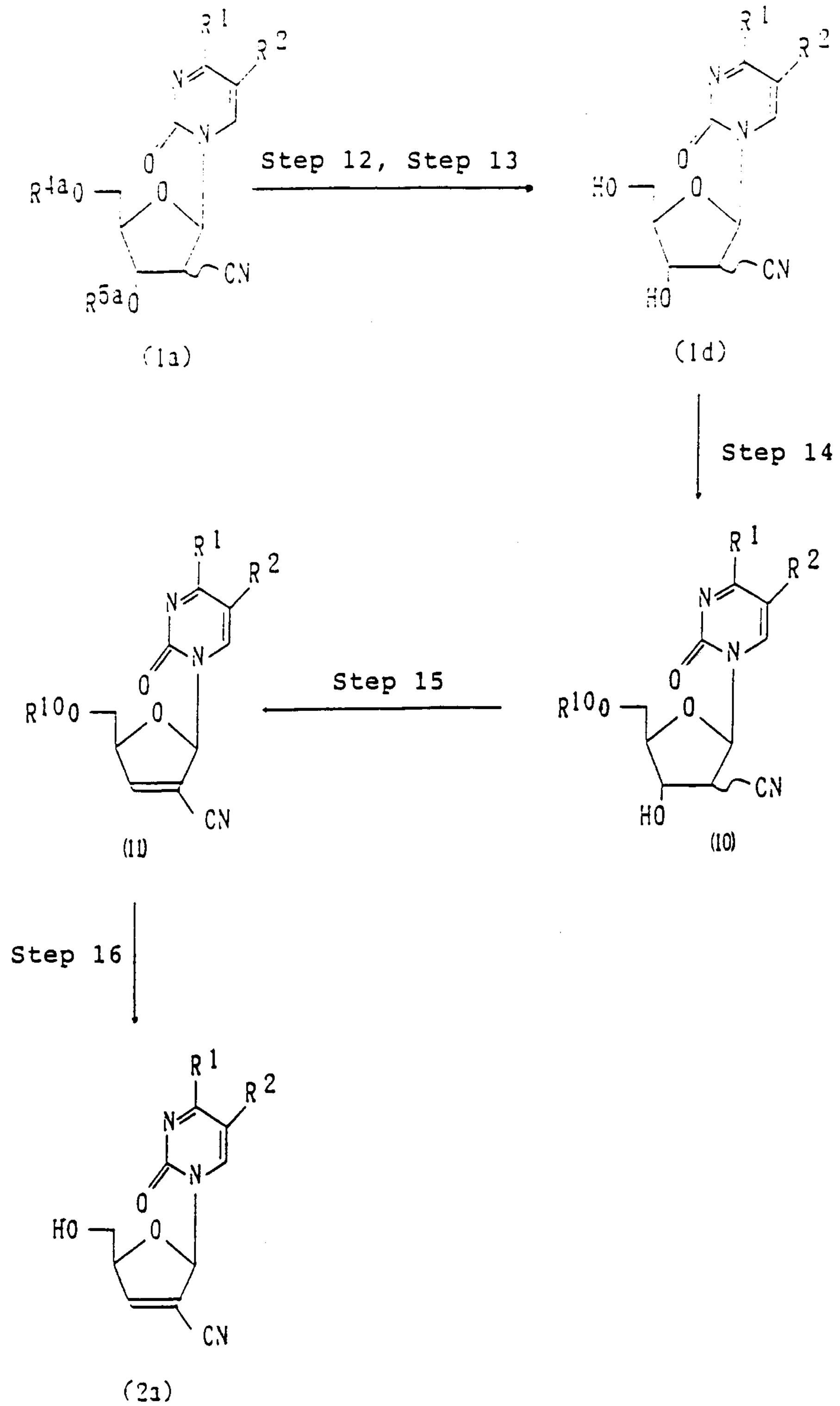


(1a) (1d) (10) (11) (2a)

Compounds having the general formulae (1) and (2) of the present invention can be prepared following the reaction steps shown in Reaction Schemes 1, 2, 3 and 4 above, using uracil or a 5-lower alkyluracil [a compound of formula (3)], known compounds which may be prepared in accordance with M. Muraoka, et al., Chem. Pharm. Bull., 18, 261 (1970).

In Reaction Schemes 1, 2, 3 and 4 above, $R^1$, $R^2$, $R^{4a}$, $R^{5a}$, $R^6$, $R^{6'}$, $R^7$, $R^{7'}$, $R^9$ and $R^{10}$ are as defined above, and X represents a halogen atom. Where $R^9$ represents an alkoxythiocarbonyl group having a $C_1$ - $C_4$ alkyl group, the alkyl group may be selected from meanings given for such groups as described hereinbefore, and is preferably methyl, and where $R^9$ represents an aryloxythiocarbonyl group having a $C_6$ - $C_{10}$ aryl, the aryl group may be selected from meanings given for such groups as described hereinbefore, and is preferably phenyl.

$R^{10}$ represents a protective group, particularly a triarylmethyl group in which the aryl moiety may be substituted. The aryl moiety may be as exemplified, such as phenyl or naphthyl, and is preferably phenyl. Suitable substituents for the aryl moiety include an alkyl group having from 1 to 4 carbon atoms, such as methyl, ethyl, propyl and butyl, an alkoxy group having from 1 to 4 carbon atoms, such as methoxy, ethoxy, propoxy and butoxy, and an acyloxy group having from 2 to 4 carbon atoms, such as acetoxy, propyloxy and butyryloxy, of which methyl and methoxy are preferred.

X represents a halogen atom, preferably chlorine or bromine.

The respective reaction steps will be explained in more detail below.

In Step 1, a compound of formula (4) is prepared by ribosylating a compound of formula (3). Ribosylation is generally carried out by methods known in the art, for example, as follows:

(i) An alcoholic solution of mercuric chloride is added to a solution of a compound of formula (3) in aqueous sodium hydroxide, thereby to provide the mercury salt of the compound of formula (3). The resulting salt is then reacted with 2',3',5'-tri-O-benzoyl-D-ribosyl chloride, a known compound, in benzene. The resulting compound i sthen allowed to react with sodium methoxide in methanol to obtain a compound of formula (4) [M. Muraoka, A. Tanaka and T. Ueda, Chem. Pharm. Bull., 18, 261 (1970)]; or

(ii) A compound of formula (3) is reacted with trimethylsilyl chloride in benzene in the presence of an organic amine such as triethylamine to obtain bis(trimethylsilyl)- uracil, which is reacted with 2',3',5'-tri-O-benzoyl-D- ribosyl chloride. The resulting compound is then allowed to react with sodium methoxide in methanol to obtain the compound of formula (4) [T. Nishimura, B. Shimizu and I. Iwai, Chem. Pharm. Bull., 11, 1470 (1963)].

In Step 2, a compound of formula (5) is prepared by substituting the carbonyl moiety at the 4-position of the compound of formula (4) with an amino group.

The substitution with an amino group is generally carried out by methods known in the art, for example, as follows:

(i) Hexamethyldisilazane and ammonium sulphate are allowed to act on a compound of formula (4) with heating in anhydrous formamide to obtain the compound of formula (5) [Nucleic Acid Chemistry, compiled by Townsend and Tipson, 227, (1978)]; or

(ii) The hydroxyl groups at the 2'-, 3'- and 5'-positions of a compound of formula (4) are first protected by acetylation or benzoylation. Thionyl chloride and anhydrous dimethylformamide, in alcohol-free chloroform, are allowed to act on the resulting compound, followed by treatment with a methanolic solution of ammonia, to obtain a compound of formula (5) [Nucleic Acid Chemistry, compiled by Townsend and Tipson, 223, (1978)]; or

(iii) The hydroxyl groups at the 2'-, 3'- and 5'-positions of a compound of formula (4) are first protected by acetylation or benzoylation. The protected compound is then reacted with diphosphorus pentasulphide in pyridine to obtain a 4-thio compound. The resulting compound is then reacted with a lower alkyl iodide, such as methyl iodide or ethyl iodide, and an alkali metal hydroxide, such as sodium hydroxide, to obtain a 4-alkylthio compound, as an intermediate compound. This 4-alkylthio compound is further treated with liquid ammonia to obtain a compound of formula (5) [J. J. Fox, N. Miller and I. Wenpen, Journal of Medicinal Chemistry, 9, 101 (1966)].

In Step 3, a compound of formula

$X\text{-}R^6R^7Si\text{-}O\text{-}SiR^{6'}R^{7'}\text{-}X$

is allowed to act on and to protect the 3'- and 5'-positions of a compound of formula (4) to obtain a compound of formula (5). This step is carried out by known methods [M. J. Robins, J. S. Wilson, L. Sawyer and M.N.G. James, Can. J. Chem., 61, 1911, (1983)].

Suitable solvents for use in this Step are preferably basic solvents, such as pyridine. The reaction is carried out at a temperature of -10 to 100 °C, preferably 0 to 50 °C.

While the reaction time varies depending on the compound and reaction temperature employed, usual reaction times are from 1 hour to 30 hours, preferably from 1 hour to 5 hours.

After completion of the reaction, for example, the solvent is distilled off, and the reaction mixture is poured in water. The resulting mixture is extracted with a water-immiscible solvent, such as benzene, ether

or ethyl acetate, and the solvent is distilled off from the extract to obtain a compound. The thus obtained compound is used as such in the subsequent step. If desired, the compound can be purified by isolation, such as by means of various chromatographic procedures or recrystallisation.

In Step 3', the amino group at the 4-position of a compound of formula (5) is acylated. The acylated compound is then allowed to react, in a similar manner to that of Step (3), with a compound of formula

$X\text{-}R^6R^7Si\text{-}O\text{-}SiR^{6'}R^{7'}\text{-}X$

to protect the 3'- and 5'-positions and to yield a compound of formula (6).

The acylation of the amino group at the 4-position is carried out by a method generally employed in the art. For example, in the case of acylation with an aliphatic acyl or aromatic acyl group, the reactive derivative of the corresponding carboxylic acid, such as an acid halide or an acid anhydride, is allowed to react with the amino group directly, or the corresponding carboxylic acid is allowed to react with the amino group in the presence of a condensing agent; or, in the case of alkoxycarbonyl, alkenyloxycarbonyl or aralkyloxycarbonyl, a halogenoformic acid ester having the corresponding alkoxy, alkenyloxy or aralkyloxy group, or dialkyl dicarbonate, dialkenyl dicarbonate or diaralkyl dicarbonate having the corresponding alkyl, alkenyl or aralkyl group is allowed to react with the amino group.

Suitable acid halides for use in accordance with this method include acid chlorides and acid bromides.

Suitable condensing agents for use in accordance with this method include:
N,N'-dicyclohexylcarbodiimide; 1,1'-oxalyldiimidazole;
2,2'-dipyridyldisulphide; N,N'-disuccinimidyl carbonate;
N,N'-bis(2-oxo-3-oxazolydinyl)phosphinic chloride;
N,N'-carbodiimidazole; N,N'-disuccinimidyl oxalate;
N,N'-diphthalimide oxalate; N,N'-bis(norbornenylsuccinimidyl)oxalate; 1,1'-bis(benzotriazolyl)oxalate;
1,1'-bis(6-chlorobenzotriazolyl)oxalate; and
1,1'-bis(6-trifluoromethylbenzotriazolyl)oxalate.

Solvents are not particularly limited, but should not inhibit the reaction. Suitable solvents include: aromatic hydrocarbons, such as benzene, toluene and xylene; ethers, such as diethyl ether, diisopropyl ether, tetrahydrofuran, dioxane, dimethoxyethane and diethylene glycol diemethyl ether; alcohols such as methanol, ethanol, n-propanol, isopropanol, n-butanol, isobutanol, t-butanol, isoamyl alcohol, diethylene glycol, glycerol, octanol, cyclohexanol and methyl cellosolve; ketones, such as acetone, methyl ethyl ketone, methyl isobutyl ketone, isophorone and cyclohexanone; nitriles, such as acetonitrile and isobutyronitrile; amides such as formamide, dimethylformamide, dimethylacetamide and hexamethylphosphorotriamide; sulphoxides such as dimethyl sulphoxide and sulpholane; and mixtures thereof with water.

Preferred solvents are: aromatic hydrocarbons, such as benzene, toluene and xylene; ethers, such as diethyl ether, diisopropyl ether, tetrahydrofuran, dioxane, dimethoxyethane and diethylene glycol dimethyl ether; alcohols, such as methanol, ethanol, n-propanol, isopropanol, n-butanol, isobutanol, t-butanol, isoamyl alcohol, diethylene glycol, glycerol, octanol, cyclohexanol and methyl cellosolve; ketones, such as acetone, methyl ethyl ketone, methyl isobutyl ketone, isophorone and cyclohexanone; nitriles, such as acetonitrile and isobutyronitrile; amides, such as formamide, dimethylformamide, dimethylacetamide and hexamethylphosphorotriamide; sulphoxides, such as dimethyl sulphoxide and sulpholane; and mixtures of these organic solvents and water.

The reaction is carried out at a temperature of from 0 °C to 150 °C, preferably 0 °C to 100 °C. While the reaction time varies, depending on such parameters as the compound and reaction temperature, it usually varies from 1 hour to 30 hours, preferably from 2 hours to 5 hours.

After completion of the rection, for example, the solvent is distilled off, and the reaction mixture is poured into water. The resulting mixture is extracted with a water-immiscible solvent, such as benzene, ether or ethyl acetate, and the solvent is distilled off from the extract to obtain a compound. The thus obtained compound is usually used, as such, in the subsequent step. If desired, the compound can be purified by isolation, for example by means of various chromatographic procedures or by recrystallisation.

In Step 4, a compound of formula (7) is prepared by substituting the hydroxyl group at the 2'-position of a compound of formula (5) with a thiocarbonyl group. This is effected by the treatment of a compound of formula (6) with a thiocarbonyl-introducing agent in an inert solvent.

Solvents are not particularly limited, but should not inhibit the reaction. Suitable solvents include: amides, such as dimethylformamide and dimethylacetamide; sulphoxides such as dimethyl sulphoxide; and nitriles, such as acetonitrile. The preferred solvent is acetonitrile.

The thiocarbonyl-introducing agent is not particularly limited but, if it is a hydroxyl group thiocarbonylating reagent, then it typically includes: lower alkoxycarbonyl halides, such as methoxythiocarbonyl chloride and ethoxythiocarbonyl chloride; and arylthiocarbonyl halides, such as phenoxythiocarbonyl chloride and naphthoxythiocarbonyl chloride.

The reaction is carried out at a temperature of from -20 °C to 50 °C, preferably at a temperature of from -10 °C to 10 °C. While the reaction time varies depending on such parameters as the compound and reaction temperature, it is usually from 1 hour to 30 hours, preferably from 2 hours to 5 hours.

For enhanced performance of the reaction, organic amines, such as 4,4-dimethylaminopyridine and triethylamine, can be used.

After completion of the reaction, the desired compound can be obtained by conventional methods. For example, the reaction mixture can be poured into water, the resulting mixture extracted with a water-immiscible solvent, such as benzene, ether or ethyl acetate, and the solvent distilled off from the extract to obtain the desired compound. The thus obtained compound is usually used as such in the subsequent step. If desired, the compound can be purified by isolation, for example by means of various chromatographic procedures or by recrystallisation.

In Step 5, a compound of formula (1a) is prepared by by treating a compound of formula (7) (obtained in accordance with Step 4 above) with a reducing agent and a nitrile-introducing agent in an inert solvent. Compounds of formula (1a) form a subgroup of the compounds of formula (1), $R^4$ and $R^5$ together representing a group of the formula:

$-R^6R^7Si-O-SiR^{6'}R^{7'}-$

and $R^3$ representing a hydrogen atom.

Solvents are not particularly limited, but should not inhibit the reaction. Suitable solvents include: aliphatic hydrocarbons, such as hexane, heptane, ligroin and petroleum ether; aromatic hydrocarbons, such as benzene, toluene and xylene; ethers, such as diethyl ether, diisopropyl ether, tetrahydrofuran, dioxane, dimethoxyethane and diethylene glycol diemethyl ether; and aromatic hydrocarbons, such as benzene, toluene and xylene. Preferred solvents are aromatic hydrocarbons, such as benzene and toluene.

Suitable nitrile-introducing agents for use in this Step preferably include alkylisonitriles, such as t-butylisonitrile, while suitable reducing reagents are preferably trialkyl-tin hydrides having from 1 to 4 carbon atoms, such as tributyl-tin hydride.

The reaction is carried out at a temperature of from 50 °C to 250 °C, preferably 80 °C to 150 °C. While the reaction time varies depending on such parameters as the compound and reaction temperature, it is usually from 30 minutes to 12 hours, preferably from 1 hour to 5 hours.

For enhanced performance of the reaction, a radical initiator, such as azoisobutyronitrile, can be used as a catalyst.

The compound of formula (1a) obtained by this step consists of a mixture of compounds wherein the nitrile group is in either the $\alpha$- or $\beta$-coordination, respectively. In order to resolve the mixture, the compounds prepared by this Step can be subjected, for example, depending on the purpose, to adsorption or ion exchange chromatography using various carriers, such as an activated carbon and silica gel, gel filtration using a Sephadex column, or recrystallisation using an organic solvent, such as ether, ethyl acetate or chloroform.

In Step 6, a compound of formula (8) is prepared by oxidising the hydroxyl group at the 2'-position of a compound of formula (6). Such oxidisation can be effected by known methods. [F. Hansske et al., Tetrahedron Letters, 40, 125, (1984)].

Solvents are not particularly limited, but should not inhibit the reaction and can dissolve the starting materials to some degree. Suitable solvents include: aromatic hydrocarbons, such as benzene, toluene and xylene; halogenated hydrocarbons, such as methylene chloride and chloroform; ethers, such as ether, tetrahydrofuran, dioxane and dimethoxyethane; amides, such as diemethylformamide, dimethylacetamide and hexamethylphosphorotriamide; sulphoxides, such as dimethyl sulphoxide; ketones, such as acetone and methyl ethyl ketone; and nitriles, such as acetonitrile. Preferred solvents are the halogenated hydrocarbons, such as methylene chloride and chloroform.

The reaction is carried out at a temperature of 0 °C to 100 °C, preferably 10 °C to 40 °C. While the reaction time varies depending on such parameters as the compound and reaction temperature, it is usually from 10 minutes to 12 hours, preferably 30 minutes to 3 hours.

The above oxidation reaction can be accelerated by the addition of an interlayer moving catalyst, such as triethylbenzylammonium chloride or tributylbenzylammonium bromide.

The resulting compound of formula (8) prepared by this step can be collected, separated and purified by combining various suitable methods. For example, the reaction mixture can be poured into water, the resulting mixture extracted with a water-immiscible solvent, such as benzene, ether or ethyl acetate, and the solvent distilled off from the extract to obtain the the target compound of formula (8). If necessary, the thus obtained compound can further be subjected, for example, to adsorption or ion exchange chromatography using various carriers, such as an activated carbon and silica gel, gel filtration using a Sephadex column or recrystallisation using an organic solvent, such as ether, ethyl acetate or chloroform.

In Step 7, a compound of formula (1b) is prepared by allowing a compound of formula (8) to react with a cyanide-introducing agent in an inert solvent in the presence of a base.

Solvents are not particularly limited, but should not inhibit the reaction. Suitable solvents include: a mixed solvent of an aliphatic hydrocarbon, such as hexane, heptane, ligroin and petroleum ether with water; a mixed solvent of an aromatic hydrocarbon, such as benzene, toluene and xylene with water; and, a mixed solvent of an ether, such as diethyl ether, diisopropyl ether, tetrahydrofuran, dioxane, dimethoxyethane and diethylene glycol diemethyl ether with water. Preferred is a mixed solvent of an ether with water.

Suitable bases for use in accordance with this Step are not particularly limited, and may include organic bases and inorganic bases, for example, alkali metal hydroxides, such as sodium hydroxide and potassium hydroxide, and alkali metal carbonates, such as sodium carbonate and potassium carbonate. An alkali metal hydrogencarbonate is preferred.

The cyanide compound for use in accordance with this Step is not particularly limited, provided that it is soluble in water to form a cyano ion, and is preferred are cyanides of alkali metals, such as sodium cyanide and potassium cyanide.

The reaction is carried out at a temperature of from 0 °C to 100 °C, preferably 10 °C to 40 °C. While the reaction time varies depending on such parameters as the compound and reaction temperature, it is usually from 30 minutes to 96 hours, preferably 5 hours to 24 hours.

The resulting compound of formula (1b) prepared by this step can be collected, separated and purified by combining various suitable methods. For example, the reaction mixture can be poured into water, the resulting mixture extracted with a water-immiscible solvent, such as benzene, ether or ethyl acetate, and the solvent distilled off from the extract to obtain the the target compound of formula (1b). If necessary, the thus obtained compound can further be subjected, for example, to adsorption or ion exchange chromatography using various carriers, such as an activated carbon and silica gel, gel filtration using a Sephadex column or recrystallisation using an organic solvent, such as ether, ethyl acetate or chloroform.

The compound of formula (1b) obtained by this step consists of a mixture of compounds wherein the nitrile group is in either the α- or β-coordination, respectively. In order to resolve the mixture, the compounds prepared by this Step can be subjected, for example, depending on the purpose, to adsorption or ion exchange chromatography using various carriers, such as an activated carbon and silica gel, gel filtration using a Sephadex column, or recrystallisation using an organic solvent, such as ether, ethyl acetate or chloroform.

In Step 8, the hydroxyl group at the 2'-position of a compound of formula (1b) is substituted with a thiocarbonyl group to yield a compound of formula (9). This is effected by treating a compound of formula (1b) with a thiocarbonyl-introducing reagent in an inert solvent in a similar manner to that described in Step 4.

In Step 9, the thiocarbonyloxy group at the 2'-position of a compound of formula (9) is eliminated by reduction to yield a compound of formula (1a).

Solvents are not particularly limited, but should not inhibit the reaction. Suitable solvents include: aliphatic hydrocarbons, such as hexane, heptane, ligroin and petroleum ether; aromatic hydrocarbons, such as benzene, toluene and xylene; ethers, such as diethyl ether, diisopropyl ether, tetrahydrofuran, dioxane, dimethoxyethane and diethylene glycol dimethyl ether; and aromatic hydrocarbons, such as benzene and toluene. Preferred solvents are aromatic hydrocarbons, such as benzene and toluene.

Reducing reagents preferably include trialkyltin hydrides, such as tributyltin hydride.

The reaction is carried out at a temperature of from 50 °C to 250 °C, preferably at the boiling point of the solvent employed. While the reaction time varies depending on such parameters as the compound and reaction temperature, it is usually from 30 minutes to 10 hours, preferably 30 minutes to 3 hours.

In order to enhance the reaction, a radical initiator, such as azoisobutyronitrile, can be used as a catalyst.

The target compound prepared by this step can be collected, separated and purified by combining various suitable methods. For example, the reaction mixture can be poured into water, the resulting mixture extracted with a water-immiscible solvent, such as benzene, ether or ethyl acetate, and the solvent distilled off from the extract to obtain the the target compound of formula (1a). If necessary, the thus obtained

compound can further be subjected, for example, to adsorption or ion exchange chromatography using various carriers, such as an activated carbon and silica gel, gel filtration using a Sephadex column or recrystallisation using an organic solvent, such as ether, ethyl acetate or chloroform.

The compound of formula (1a) obtained by this step consists of a mixture of compounds wherein the nitrile group is in either the $\alpha$- or $\beta$-coordination, respectively. In order to resolve the mixture, the compounds prepared by this Step can be subjected, for example, depending on the purpose, to adsorption or ion exchange chromatography using various carriers, such as an activated carbon and silica gel, gel filtration using a Sephadex column, or recrystallisation using an organic solvent, such as ether, ethyl acetate or chloroform.

In Steps 10 and 12, compounds of formulae (1c) and (1d) are prepared by the elimination of the protecting groups represented by $R^{4a}$ and $R^{5a}$ in compounds of formulae (1b) and (1a). The reaction is carried out in an inert solvent to remove the substituent on the amino group, as necessary.

Solvents are not particularly limited, but should not inhibit the reaction. Preferable solvents for use in these Steps include ethers, such as tetrahydrofuran and dioxane. Suitable reagents for effecting the elimination are not particularly restricted, provided that it is usually used for eliminating a silyl group. Suitable reagents include those which form a fluorine anion, such as tetrabutylammonium fluoride.

The reaction is carried out at a temperature of from 0°C to 40°C, preferably at room temperature. While the reaction time varies depending on the reaction temperature, it is usually from 10 minutes to 24 hours, preferably 1 hour to 5 hours.

The target compound prepared by this step can be collected, separated and purified by combining various suitable methods. For example, the reaction mixture can be poured into water, the resulting mixture extracted with a water-immiscible solvent, such as benzene, ether or ethyl acetate, and the solvent distilled off from the extract to obtain the the target compound of formula (8). If necessary, the thus obtained compound can further be subjected, for example, to adsorption or ion exchange chromatography using various carriers, such as an activated carbon and silica gel, gel filtration using a Sephadex column or recrystallisation using an organic solvent, such as ether, ethyl acetate or chloroform.

In the case where $R^2$ is a substituted amino group, it can sometimes be eliminated simultaneously.

In Steps 11 and 13, compounds of formulae (1c) and (1d) of the present invention are prepared, if desired, by the elimination of the substituent at $R^2$. This is suitably effected by the action of an eliminating agent in an inert solvent, thereby to eliminate the protective group.

While the method of elimination will vary according to the protective moiety, it is usually carried out by a method known in the art. For example:

a) When the protective moiety is an aliphatic acyl, aromatic acyl or alkyloxycarbonyl group, it can suitably be removed by treating with an acid in the presence or absence of a solvent.

Suitable acids include hydrochloric acid, acetic acid, sulphuric acid, phosphoric acid and hydrobromic acid. The preferred acid is acetic acid.

Suitable solvents include: alcohols, such as methanol, ethanol, n-propanol, isopropanol, n-butanol, isobutanol, t-butanol, isoamyl alcohol, diethylene glycol, glycerol and octanol; aromatic hydrocarbons, such as benzene, toluene and xylene; ethers, such as tetrahydrofuran and dioxane; and mixtures of such organic solvents with water.

The reaction is carried out at a temperature of 0°C to 40°C, but preferably at room temperature. While the reaction time will vary depending on the reaction temperature, it is typically from 10 minutes to 24 hours, preferably from 1 hour to 5 hours.

The desired compound obtained by this elimination can be collected, separated and purified by a suitable combination of various methods. In general, the reaction mixture is distilled off and the residue is subjected, for example, to:

adsorption or ion exchange chromatography on various carriers, such as activated carbon and silica gel;
gel filtration on a Sephadex column; or
recrystallisation from an organic solvent, such as ether, ethyl acetate or chloroform.

or

b) When the protective moiety is an aralkyloxycarbonyl group, it may be eliminated by catalytic reduction.

Suitable solvents include: alcohols, such as methanol, ethanol, n-propanol, isopropanol and n-butanol; saturated hydrocarbons, such as hexane and cyclohexane; ethers, such as tetrahydrofuran and dioxane; and lower fatty acids, such as acetic acid and propionic acid. Preferred solvents are methanol, ethanol, acetic acid and propionic acid.

Preferred catalysts are platinum and palladium-on-carbon.

The reaction is carried out at a temperature of from 0°C to 40°C, preferably at room temperature.

While the reaction time will vary depending on the reaction temperature, it is typically from 10 minutes to 24 hours, preferably 1 hour to 5 hours.

The desired compound obtained by this elimination can be collected, separated and purified by a suitable combination of various methods. In general, the reaction mixture is distilled off and the residue is subjected, for example, to:
adsorption or ion exchange chromatography on various carriers, such as activated carbon and silica gel;
gel filtration on a Sephadex column; or
recrystallisation from an organic solvent, such as ether, ethyl acetate or chloroform.

In Step 14, a compound of formula (1d) is allowed to react with a protecting reagent in an inert solvent to yield a compound of formula (10).

Suitable solvents include: basic solvents, such as pyridine; and neutral solvents, such as benzene, toluene and ether.

The protecting reagent for use in accordance with this Step is not particularly limited, provided that it can be used to specifically protect only the hydroxyl group at the 5'-position. Suitable protecting reagents include triphenylchloromethane, monomethoxytrityl chloride and dimethoxytrityl chloride.

The reaction is usually carried out at a temperature of from 0°C to 100°C, preferably from -10°C to 50°C. The reaction time is usually from 30 minutes to 10 hours, preferably from 1 hour to 5 hours.

Where a neutral solvent is employed, efficiency of the reaction may be enhanced by the use of an organic amine, such as triethylamine.

The compound obtained by this Step can be collected, separated and purified by a suitable combination of various methods. For example, the reaction mixture can be poured into water, and the resulting mixture extracted with a water-immiscible solvent, such as benzene, ether or ethyl acetate, and the solvent distilled off from the extract to obtain the desired compound. If necessary, the thus obtained compound can be subjected, for example, to adsorption or ion exchange chromatography using various carriers, such as activated carbon and silica gel, gel filtration on a Sephadex column, or recrystallisation using an organic solvent, such as ether, ethyl acetate or chloroform.

In Step 15, a compound of formula (10) is allowed to react with a hydroxyl eliminating agent in an inert solvent to yield a compound of formula (11).

Suitable solvents include: aliphatic hydrocarbons, such as hexane, heptane, ligroin and petroleum ether; aromatic hydrocarbons, such as benzene, toluene and xylene; ethers, such as diethyl ether, diisopropyl ether, tetrahydrofuran, dioxane, dimethoxyethane and diethylene glycol dimethyl ether; ketones, such as acetone, methyl ethyl ketone, methyl isobutyl ketone, isophorone and cyclohexanone; nitro compounds, such as nitroethane and nitrobenzene; nitriles, such as acetonitrile and isobutyronitrile; amides, such as formamide, dimethylformamide, dimethylacetamide and hexamethyl phophorotriamide; and sulphoxides, such as dimethyl sulphoxide and sulpholane. Preferred solvents are aromatic hydrocarbons, such as benzene, toluene and xylene.

Suitable hydroxyl eliminating reagents include compounds having a thiocarbonyl group, such as thiocarbonyl diimidazole and phenoxythiocarbonyl chloride.

The reaction is carried out at a temperature of from -10°C to 50°C, preferably at room temperature. The reaction time is usually from 1 hour to 24 hours, preferably from 3 hours to 10 hours.

The compound obtained by this Step can be collected, separated and purified by a suitable combination of various methods. For example, the reaction mixture can be poured into water, and the resulting mixture extracted with a water-immiscible solvent, such as benzene, ether or ethyl acetate, and the solvent distilled off from the extract to obtain the desired compound. If necessary, the thus obtained compound can be subjected, for example, to adsorption or ion exchange chromatography using various carriers, such as activated carbon and silica gel, gel filtration on a Sephadex column, or recrystallisation using an organic solvent, such as ether, ethyl acetate or chloroform.

In Step 16, a compound of formula (2a) of the present invention is prepared by allowing a compound of formula (11) to react with a hydroxyl group deprotecting agent in an inert solvent.

While the method of elimination will vary according to the protective moiety, it is usually carried out by a method known in the art. Where the protecting moiety is a triarylmethyl (a preferred protective group), it is carried out, for example, by use of an acid in an inert solvent.

Solvents for use in accordance with the present Step are not particularly limited, provided that they do not participate in the reaction. Preferred sovents include: alcohols, such as methanol and ethanol; ethers, such as tetrahydrofuran and dioxane; and mixtures of such organic solvents with water.

The hydroxyl group deprotecting agent is usually an acid. The acid is not particularly limited, provided that it is used as a Brønsted acid. Preferred acids include: inorganic acids, such as hydrochloric acid and sulphuric acid; and organic acids, such as acetic acid and p-toluenesulphonic acid. In addition, a strong

acidic cation exchange resin, such as Dowex 50W can also be used.

The reaction is usually carried out at a temperature of from 0°C to 50°C, preferably at room temperature. While the reaction time will vary on such parameters as the starting materials and type of acids, it is usually from 10 minutes to 18 hours, preferably from 30 minutes to 5 hours.

The compound obtained by this Step can be collected, separated and purified by a suitable combination of various methods. For example, the reaction mixture can be poured into water, and the resulting mixture extracted with a water-immiscible solvent, such as benzene, ether or ethyl acetate, and the solvent distilled off from the extract to obtain the desired compound. If necessary, the thus obtained compound can be subjected, for example, to adsorption or ion exchange chromatography using various carriers, such as activated carbon and silica gel, gel filtration on a Sephadex column, or recrystallisation using an organic solvent, such as ether, ethyl acetate or chloroform.

The pyrimidine nucleoside derivatives of the present invention can be administered to warm-blooded animals, including humans. Suitable administration forms include: intravenous injections; subcutaneous injections; intramuscular injections and suppositories; and, for oral administration: tablets; capsules; powders and granules.

The pyrimidine nucleoside derivatives of the present invention can be made up into the desired administration forms using conventional methods. Accordingly, the present invention includes pharmaceutical preparations and compositions containing pharmaceutically acceptable pyrimidine nucleoside derivatives as defined herein.

In addition, the compounds of the present invention can be used in combination with other antitumour agents, such as nitrosourea drugs, e.g. 5Fu, AraC, ACNU and BCNU, cisplatin, daunomycin, adriamycin, mitomycin C or etoposide.

Compositions for injection may be provided in unit dosage ampoules or multiple-dosage containers. The composition may contain additives, such as suspending agents, stabilisers and dispersing agents, and is usually a powder which is redissolved before use in an appropriate solvent, such as a sterilised aqueous medium containing no pyrogenic material. Such formulations can be prepared, for example, by dissolving the pyrimidine nucleoside derivative in acetone, pouring into vials and freeze-drying after the addition of water.

The compositions for oral administration may be provided in the form of tablets, capsules, powders, granules or syrups containing suitable amounts of the pyrimidine nucleoside derivatives for administration.

While the dose for an adult will vary depending on such factors as the nature of the condition to be treated, the administration route, the number of dosages and the administration period, preparations may suitably be administered in an amount of 0.01 to 5 g per day once or in several doses.

The present invention will now be described in further detail by way of the following Examples, Reference Examples and Preparation Examples. In the Examples, TIPDS represents the (1,1,3,3-tetraisopropyldisilox-1,3-diyl) moiety.

## EXAMPLE 1

### 1-[2'-Cyano-3',5,-O-(1,1,3,3-tetraisopropyldisilox-1,3-diyl)-β-D-ribofuranosyl]thymine

997 mg 1-(3,5-O-TIPDS-β-D-erythro-pentofuran-2-urosyl)thymine was dissolved in 15 ml of ether-water (2 : 1), and 196 mg of sodium cyanide and 336 mg of sodium hydrogencarbonate were added to the resulting solution, which was then stirred at room temperature for 36 hours. After completion of the reaction, ethyl acetate was added to the reaction mixture, and the mixture was washed three times with water. The ethyl acetate layer was dried over anhydrous sodium sulphate and the solvent removed by evaporation. The residue was purified by column chromatography using a silica gel column (ϕ2.4 x 9.5 cm) [eluted with hexane/ethyl acetate (2 : 1)] to obtain 1.03 g (97.5%) of the title compound as a white foam.

Nuclear Magnetic Resonance spectrum ($CDCl_3$) δppm:

9.20 and 8.52 (1H, broad singlet);
7.43 and 7.36 (1H, doublet, J = 6.8 Hz);
6.22 and 6.00 (1H, singlet);
5.08 (1H, broad singlet);
4.32-3.94 (4H, multiplet);
1.92 (3H, doublet, J = 1.7 Hz);
1.12-1.07 (28H, multiplet).

EXAMPLE 2

1-(2'-Cyano-2'-deoxy-3',5,-O-TIPDS-β-D-arabinofuranosyl)thymine

100 mg of the title compound of Example 1 and 10 mg of 4,4-dimethylaminopyridine (hereinafter abbreviated as DMAP) were dissolved in 2 ml of anhydrous acetonitrile. 39 µl of phenoxycarbonyl chloride and 40 µl of triethylamine were added to the resulting solution at 0 °C in an argon gas stream, and the solution was then stirred for 3 hours. After completion of the reaction, ethyl acetate was added to the reaction mixture and the mixture was washed three times with water. The mixture was dried over anhydrous sodium sulphate, and the solvent was removed by evaporation. The residue was purified by column chromatography using a silica gel column (ϕ1.6 x 10 cm) [eluted with methanol/chloroform (1 : 99)] to obtain 71 mg (73.3%) of the title compound as a yellowish white foam.
Nuclear Magnetic Resonance spectrum ($CDCl_3$) δppm:

7.36 (1H, doublet, J = 1.2 Hz);
6.28 (1H, doublet, J = 7.3 Hz);
4.67 (1H, doublet of doublets, J = 8.3, 9.3 Hz);
4.17 (1H, doublet of doublets, J = 2.2, 13.2 Hz);
4.04 (1H, doublet of doublets, J = 2.9, 13.2 Hz);
3.78 (1H, double of double doublets, J = 2.2, 2.9, 8.3 Hz);
3.58 (1H, doublet of doublets, J = 7.3, 9.3 Hz);
1.94 (1H, doublet, J = 1.2 Hz);
1.15-1.04 (28H, multiplet).

EXAMPLE 3

1-(2'-Cyano-2'-deoxy-β-D-arabinofuranosyl)thymine

178 mg of the compound of Example 2 were dissolved in 3 ml of anhydrous tetrahydrofuran (THF), and 20 µl of acetic acid and 0.70 ml of a solution of tetrabutylammonium fluoride in 1M-THF were added dropwise at 0 °C in an argon gas stream to the resulting solution, which was then stirred for 1.5 hours. After completion of the reaction, the reaction mixture was concentrated, purified by column chromatography using a silica gel column (ϕ1.8 x 8 cm) [eluted with ethanol/chloroform (8-10 : 92-90)] and crystallised from ether and ethanol to obtain 27 mg of the title compound as white crystals.
Nuclear Magnetic Resonance spectrum ($CDCl_3$) δppm:

11.49 (1H, singlet);
7.85 (1H, doublet, J = 1.1 Hz);
6.25 (1H, doublet, J = 6.0 Hz);
6.20 (1H, doublet, J = 7.1 Hz);
5.30 (1H, triplet, J = 4.9 Hz);
4.47 (1H, double of double doublets, J = 6.0, 8.2, 8.8 Hz);
3.89 (1H, doublet of doublets, J = 7.1, 8.8 Hz);
3.74 (1H, double of double doublets, J = 2.2, 3.3, 8.2 Hz);
3.62 (1H, double of double doublets, J = 3.3, 4.9, 11.1 Hz);
1.78 (3H, doublet, J = 1.1 Hz).

EXAMPLE 4

1-(2'-Cyano-2'-deoxy-3',5'-O-TIPDS-β-D-arabinofuranosyl)-$N^4$-benzoylcytosine

The procedure of Example 1 was repeated, but using 294 mg of $N^4$-benzoyl-1-(3,5-O-TIPDS-β-D-erythropentofuran-2-urosyl)cytosine. Using the resulting crude product, the procedure of Example 2 was repeated to obtain 174 mg (49.1%) of the title compound as a yellowish white solid.
Nuclear Magnetic Resonance spectrum ($CDCl_3$) δppm:

8.89 (1H, broad singlet);
8.11 (1H, doublet, J = 7.7 Hz);
7.93-7.45 (5H, multiplet);
7.67 (1H, doublet, J = 7.7 Hz);
6.36 (1H, doublet, J = 6.6 Hz);

4.67 (1H, triplet, J = 8.1 Hz);
4.18 (1H, doublet of doublets, J = 2.9, 13.2 Hz);
4.10 (1H, doublet of doublets, J = 2.9, 13.2 Hz);
3.91 (1H, double of double doublets, J = 2.9, 2.9, 8.1 Hz);
3.75 (1H, doublet of doublets, J = 6.6, 8.1 Hz);
1.15-1.04 (28H, multiplet).

EXAMPLE 5

1-(2'-Cyano-2'-deoxy-β-D-arabinofuranosyl)-$N^4$-benzoylcytosine

The procedure of Example 3 was repeated using 100 mg of the title compound of Example 4. Crystallisation from methanol yielded 25 mg of the title compound as white crystals.
Nuclear Magnetic Resonance spectrum (hexadeuterated dimethyl sulphoxide) δppm:
11.34 (1H, broad singlet);
8.45 (1H, doublet, J = 7.7 Hz);
8.00 (2H, multiplet);
7.66-7.49 (3H, multiplet);
7.42 (1H, doublet, J = 7.7 Hz);
6.29 (1H, doublet, J = 5.5 Hz);
6.25 (1H, doublet, J = 7.1 Hz);
5.28 (1H, broad singlet);
4.47 (1H, double of double doublets; J = 5.5, 7.1, 7.7 Hz);
3.94 (1H, doublet of doublets, J = 7.1, 7.7 Hz);
3.86 (1H, double of double doublets, J = 2.5, 3.8, 7.1 Hz);
3.79 (1H, broad doublet, J = 12.5 Hz);
3.65 (1H, broad doublet, J = 12.5 Hz).

EXAMPLE 6

1-(2'-Cyano-2'-deoxy-3',5'-O-TIPDS-β-D-arabinofuranosyl)-$N^4$-acetylcytosine

The procedure of Example 4 was repeated, but using 2 g of $N^4$-acetyl-1-(3,5-O-TIPDS-β-D-erythropentofuran-2-urosyl)cytosine. After purification, the crystals obtained by evaporation of the solvent were collected by filtration with ether-hexane to yield 703 mg of the title compound as white crystals.
Nuclear Magnetic Resonance spectrum ($CDCl_3$) δppm:
9.92 (1H, broad singlet);
8.07 (1H, doublet, J = 7.7 Hz);
7.55 (1H, doublet, J = 7.7 Hz);
6.34 (1H, doublet, J = 7.0 Hz);
4.63 (1H, triplet, J = 8.8 Hz);
4.18 (1H, doublet of doublets, J = 2.4, 13.4 Hz);
4.06 (1H, doublet of doublets, J = 2.7, 13.4 Hz);
3.89 (1H, double of double doublets, J = 2.4, 2.7, 8.8 Hz);
3.72 (1H, doublet of doublets, J = 7.0, 8.8 Hz);
2.30 (3H, singlet);
1.13-1.03 (28H, multiplet).

EXAMPLE 7

1-(2'-Cyano-2'-deoxy-β-D-arabinofuranosyl)-$N^4$-acetylcytosine

The procedure of Example 3 was repeated, but using 1.07 g of the title compound of Example 6 and, after purification, the crystals obtained by evaporation of the solvent were collected by filtration with ether-hexane to yield 480 mg of the title compound as white crystals.
Nuclear Magnetic Resonance spectrum (hexadeuterated dimethyl sulphoxide) δppm:
10.97 (1H, broad singlet);
8.36 (1H, doublet, J = 7.7 Hz);

7.26 (1H, doublet, J = 7.7 Hz);
6.27 (1H, doublet, J = 6.1 Hz);
6.22 (1H, doublet, J = 7.1 Hz);
5.24 (1H, broad singlet);
4.43 (1H, double of double doublets, J = 6.1, 7.1, 7.1 Hz);
3.92 (1H, triplet, J = 7.1 Hz);
3.84 (1H, double of double doublets, J = 2.8, 3.3, 7.1 Hz);
3.76 (1H, broad doublet, J = 12.1 Hz);
3.63 (1H, broad doublet, J = 12.1 Hz);
2.11 (3H, singlet).

EXAMPLE 8

1-(2'-Cyano-2'-deoxy-β-D-arabinofuranosyl)cytosine

100 mg of the title compound of Example 7 was dissolved in 55 ml of methanol. 2.5 ml of acetic acid was added to the resulting solution, followed by refluxing with heating in an oil bath for 5 days. After completion of the reaction, the solvent was evaporated off and the residue was purified on a silica gel column (ϕ1.8 x 7 cm) [eluted with methanol/chloroform (12-15 : 88-85)] and further on HPLC (D-ODS-5.5% methanol-water) and then crystallised from ethanol-ether to yield 29 mg of the title compound as white crystals.
Nuclear Magnetic Resonance spectrum (hexadeuterated dimethyl sulphoxide) δppm:
7.83 (1H, doublet, J = 7.1 Hz);
7.27 (2H, broad singlet);
6.17 (1H, doublet, J = 6.6 Hz);
6.15 (1H, doublet, J = 7.1 Hz);
5.79 (1H, doublet, J = 7.6 Hz);
5.14 (1H, triplet, J = 4.9 Hz);
4.40 (1H, double of double doublets, J = 6.6, 7.1, 7.7 Hz);
3.77 (1H, triplet, J = 7.1 Hz);
3.74 (1H, double of double doublets, J = 2.8, 4.5, 7.7 Hz);
3.73 (1H, double of double doublets, J = 2.8, 4.9, 12.6 Hz);
3.60 (1H, double of double doublets, J = 2.8, 4.9, 12.6 Hz).

EXAMPLE 9

1-(2'-Cyano-2'-deoxy-β-D-arabinofuranosyl)cytosine. monohydrochloride

40 mg of the compound of Example 8 was dissolved in 5 ml of 3% hydrochloric acid-methanol and stirred at room temperature for 50 minutes. After completion of the reaction, crystallisation was carried out using ethanol-ether to obtain 26 mg of the title compound as white crystals.
Nuclear Magnetic Resonance spectrum (hexadeuterated dimethyl sulphoxide) δppm:
9.80 (1H, singlet);
8.75 (1H, singlet);
8.30 (1H, doublet, J = 7.7 Hz);
6.21 (1H, doublet, J = 7.2 Hz);
6.12 (1H, doublet, J = 7.7 Hz);
4.43 (1H, doublet of doublets, J = 7.1, 7.7 Hz);
3.97 (1H, triplet, J = 7.1 Hz);
3.83 (1H, double of double doublets, J = 2.8, 3.3, 7.7 Hz);
3.76 (1H, doublet of doublets, J = 2.8, 12.6 Hz);
3.62 (1H, doublet of doublets, J = 3.8, 12.6 Hz).

EXAMPLE 10

1-(2'-Cyano-2'-deoxy-3',5'-O-TIPDS-β-D-ribofuranosyl)thymine

400 mg of 3',5'-O-TIPDS-2'-O-phenoxythiocarbonylthymidine was suspended in 4 ml of anhydrous toluene, and 1.98 ml of t-butylisonitrile was added to the resulting suspension, followed by heating in an oil bath at 100 °C in an argon gas stream. 4 ml of a solution of azoisobutyronitrile (50 mg) and tributyltin hydride (0.25 ml) in toluene was added dropwise to the resulting mixture over one hour using a syringe pump. Three hours after completion of the dropwise addition, 0.25 ml of tributyltin hydride was added to the mixture, which was then stirred for 19 hours, followed by evaporation of the solvents. The residue was purified by column chromatography using a silica gel column ( 2.2 x 8 cm) (eluted with chloroform) to obtain 70 mg of the title compound as a yellow foam.
Nuclear Magnetic Resonance spectrum ($CDCl_3$) δppm:

8.57 (1H, broad singlet);
7.38 (1H, doublet, J = 1.1 Hz);
6.01 (1H, doublet, J = 2.6 Hz);
4.22-4.01 (4H, multiplet);
3.48 (1H, doublet of doublets, J = 2.6, 4.8 Hz);
1.90 (3H, doublet, J = 1.1 Hz);
1.10-1.01 (28H, multiplet).

EXAMPLE 11

1-(2'-Cyano-2'-deoxy-β-D-ribofuranosyl)thymine

The procedure of Example 3 was repeated, but using 70 mg of the title compound of Example 10 and, after purification, the solid obtained by evaporating the solvent was collected by filtration with ether to obtain 17 mg of the title compound as a yellowish white solid.
Nuclear Magnetic Resonance spectrum (hexadeuterated dimethyl sulphoxide) δppm:

11.49 (1H, broad singlet);
7.64 (1H, doublet, J = 1.1 Hz);
6.32 (1H, doublet, J = 5.5 Hz);
6.27 (1H, doublet, J = 8.2 Hz);
5.22 (1H, broad singlet);
4.37 (1H, double of double doublets, J = 2.8, 5.5, 5.5 Hz);
3.93 (1H, multiplet);
3.75 (1H, doublet of doublets, J = 5.5, 8.2 Hz);
3.66-3.51 (2H, multiplet);
1.78 (3H, doublet, J = 1.1 Hz).

EXAMPLE 12

1-(2'-Cyano-2',3'-deoxy-2',3'-didehydro-β-D-ribofuranosyl)thymine

112 mg of the title compound of Preparative Example 3 was dissolved in 3 ml of acetic acid, and the resulting solution was stirred at room temperature for 1 hour. After completion of the reaction, the solvent was evaporated and the residue was purified on a silica gel column (φ1.6 x 8.5 cm) (eluted with ethanol/chloroform = 8 : 92). The solvent was evaporated and the precipitated crystals were collected by filtration using ether-hexane to obtain 22 mg of the title compound as crystals.
Nuclear Magnetic Resonance spectrum ($CDCl_3$) δppm:

11.53 (1H, broad singlet);
7.81 (1H, doublet, J = 1.1 Hz);
7.63 (1H, doublet, J = 1.7 Hz);
7.02 (1H, doublet of doublets, J = 1.7, 3.9 Hz);
5.33 (1H, triplet, J = 4.9 Hz);
5.05 (1H, double of double doublets, J = 2.8, 2.8, 3.9 Hz);
3.74 (1H, double of double doublets, J = 2.8, 4.9, 12.6 Hz);
3.67 (1H, double of double doublets, J = 2.8, 4.9, 12.6 Hz);

1.75 (3H, doublet, J = 1.1 Hz).

EXAMPLE 13

1-(2'-Cyano-2',3'-dideoxy-2',3'-didehydro-β-D-arabinofuranosyl)-$N^4$-acetylcytosine

The procedure of Example 12 was repeated, but using 70 mg of the title compound of Preparative Example 4. After purification on a silica gel column (ϕ1.8 x 7 cm) (eluted with methanol/chloroform = 10 : 90), crystallisation from ethanol-ether was carried out to obtain 14 mg of the title compound as crystals.
Nuclear Magnetic Resonance spectrum (hexadeuterated dimethyl sulphoxide) δppm:

11.02 (1H, singlet);
8.33 (1H, doublet, J = 7.1 Hz);
7.65 (1H, triplet, J = 1.7 Hz);
7.24 (1H, doublet, J = 7.1 Hz);
7.12 (1H, doublet of doublets, J = 1.7, 3.3 Hz);
5.30 (1H, triplet, J = 4.9 Hz);
5.12 (1H, multiplet);
3.74 (1H, doublet of doublets, J = 3.3, 12.6 Hz);
3.67 (1H, doublet of doublets, J = 3.3, 12.6 Hz);
2.12 (3H, singlet).

EXAMPLE 14

$N^4$-Benzyloxycarbonylcytidine

4.86 g of cytidine was dissolved, as much as possible, in pyridine, and the resulting solution was twice subjected to azeotropic distillation to remove the moisture content therefrom. 100 ml of pyridinewas added to the residue, and 12.6 ml of trimethylchlorosilane was added to the resulting mixture with ice-cooling, followed by stirring for 30 minutes. 49 ml of carbobenzoxychloride (30 to 35% toluene solution) was added dropwise to the resulting mixture. The mixture was then stirred until room temperature was reached, and then left to stand overnight. 40 ml of water was added to the mixture, and the resulting mixture was stirred for 1.5 hours. Methylene chloride was added, and the organic layer was separated and washed with saturated aqueous sodium chloride. The organic layer was then dried over anhydrous magnesium sulphate and the solvent evaporated. The residue was then subjected three times to azeotropic distillation with toluene and ethanol to obtain 6.13 g of the title compound as a crystalline residue.
Nuclear Magnetic Resonance spectrum (270M Hz in hexadeuterated dimethyl sulphoxide) δppm:

8.40 (1H, doublet, J = 7.3 Hz);
7.31-7.55 (5H, multiplet);
7.02 (1H, doublet, J = 7.3 Hz);
5.77 (1H, doublet, J = 2.4 Hz);
5.19 (2H, singlet);
3.88-3.99 (3H, multiplet).

EXAMPLE 15

3',5'-O-TIPDS-$N^4$-benzyloxycarbonylcytidine

6.0 g of the title compound of Example 14 was dissolved in pyridine, and the resulting solution was twice subjected to azeotropic distillation to remove the moisture content therefrom. The residue was dissolved in 200 ml of pyridine, and 5.09 ml of 1,3-dichloro-1,1,3,3-tetraisopropyldisiloxane was added, followed by stirring at room temperature. The mixture was left to stand for 2 days, after which the solvents were evaporated. The residue was dissolved in methylene chloride, and the solution was washed successively with water, 0.5N hydrochloric acid, saturated aqueous sodium chloride, saturated aqueous sodium hydrogencarbonate and saturated aqueous sodium chloride. The residue was dried over anhydrous sodium sulphate, and the solvent was evaporated to obtain 10.23 g of the title compound.
Nuclear Magnetic Resonance spectrum (270M Hz in hexadeuterated dimethyl sulphoxide) δppm:

10.83 (1H, broad singlet);
8.12 (1H, doublet, J = 7.8 Hz);

7.32-7.43 (5H, multiplet);
7.03 (1H, doublet, J = 7.3 Hz);
5.59 (1H, singlet);
5.19 (1H, singlet);
3.91-4.24 (5H, multiplet);
0.80-1.14 (28H, multiplet).

EXAMPLE 16

$N^4$-Benzyloxycarbonyl-1-(3,5-O-TIPDS-β-D-erythropentofuran-2-urosyl)cytosine

13.16 g of pyridinium dichromate, 3.31 ml of acetic anhydride, 0.94 ml of pyridine and 2.5 g of celite were added to 70 ml of methylene chloride, and the resulting mixture was stirred for 40 minutes. Separately, 7.23 g of the title compound of Example 15 were dissolved in 30 ml of methylene chloride, and the resulting solution was added to the first mixture. The resulting mixture was stirred at room temperature for 5 hours, after which ethyl acetate was added, and methylene chloride was distilled off. The residue was dissolved in ethyl acetate and insolubles were filtered off. Subsequently, the filtrate was washed with 1N hydrochloric acid, saturated aqueous sodium chloride, saturated aqueous sodium hydrogencarbonate and saturated aqueous sodium chloride, and the filtrate dried over anhydrous magnesium sulphate. The solvent was distilled off and the residue was purified over silica gel chromatography (methylene chloride/methanol = 99 : 1) to obtain 2.84 g of the title compound.
Nuclear Magnetic Resonance spectrum (270M Hz in hexadeuterated dimethyl sulphoxide) δppm:
10.98 (1H, broad singlet);
8.15 (1H, doublet, J = 7.3 Hz);
7.32-7.44 (5H, multiplet);
7.09 (1H, doublet, J = 7.3 Hz);
5.49 (1H, singlet);
5.20 (2H, singlet);
5.07 (1H, doublet, J = 8.3 Hz);
3.95-4.04 (3H, multiplet);
0.92-1.12 (28H, multiplet).

EXAMPLE 17

$N^4$-Benzyloxycarbonyl-2'-cyano-3',5'-O-TIPDS-β-D-arabinofuranosyl-cytosine

2.71 g of the title compound of Example 16 was dissolved in 25 ml of tetrahydrofuran, and 13 ml of water was added thereto, followed by stirring. 436 mg of sodium cyanide was added to the resulting mixture with ice-cooling. 740 mg sodium hydrogencarbonate were then added to the mixture, followed by stirring at room temperature for 7 hours. The mixture was then left to stand in a refrigerator overnight, after which 217 mg of sodium cyanide was further added. The resulting mixture was stirred at room temperature for 8 hours and, after the solvents were distilled off, the residue was dissolved in ethyl acetate. The solution was washed three times with saturated aqueous sodium chloride and dried over anhydrous magnesium sulphate, followed by evaporation of the solvent. The residue was purified by silica gel chromatography (eluted with methylene chloride/methanol = 99.25 : 0.75) and crystallised from acetonitrile to obtain 392 mg of the title compound.
Nuclear Magnetic Resonance spectrum (270M Hz in hexadeuterated dimethyl sulphoxide) δppm:
11.00 (1H, broad singlet);
8.05 (1H, doublet, J = 7.3 Hz);
7.83 (1H, broad singlet);
7.33-7.44 (5H, multiplet);
7.13 (1H, doublet, J = 7.8 Hz);
5.93 (1H, singlet);
5.21 (2H, singlet);
3.93-4.33 (4H, multiplet);
0.96-1.07 (28H, multiplet).

EXAMPLE 18

$N^4$-Benzyloxycarbonyl-2'-cyano-deoxy-3',5'-O-TIPDS-β-D-arabinofuranosylcytosine

0.40 g of the title compound of Preparative Example 5 was dissolved in 8 ml of toluene, and 13.4 mg of α,α'-azobisisobutyronitrile and 0.20 ml of tributyltin hydride, in that order, were added to the resulting solution in a nitrogen gas stream, followed by stirring at 100 °C for 2 hours. The solvent was distilled off and the residue was purified by silica gel chromatography (eluted with methylene chloride/methanol = 99 : 1) to obtain 202 mg of the title compound.
Nuclear Magnetic Resonance spectrum (270M Hz in hexadeuterated dimethyl sulphoxide) δppm:
10.96 (1H, broad singlet);
8.02 (1H, doublet, J = 7.3 Hz);
7.32-7.44 (5H, multiplet);
7.12 (1H, doublet, J = 7.8 Hz);
6.20 (1H, doublet, J = 7.8 Hz);
5.20 (2H, singlet);
3.91-4.72 (5H, multiplet);
0.95-1.23 (28H, multiplet).

EXAMPLE 19

$N^4$-Benzyloxycarbonyl-2'-cyano-2'-deoxy-β-D-arabinofuranosylcytosine

192 mg of the title compound of Example 18 was dissolved in 5 ml of tetrahydrofuran and the resulting solution was ice-cooled in a nitrogen gas stream. A solution of 0.02 ml of acetic acid and 168 mg of tetrabutylammonium fluoride dissolved in 1.2 ml of tetrahydrofuran was then added, followed by stirring with ice-cooling for 2 hours. The solvents were distilled off and the residue was purified by silica gel chromatography (eluted with methylene chloride/methanol = 95 : 5) to obtain 94 mg of the title compound.
Nuclear Magnetic Resonance spectrum (270M Hz in hexadeuterated dimethyl sulphoxide) δppm:
10.92 (1H, broad singlet);
8.36 (1H, doublet, J = 7.3 Hz);
7.34-7.44 (5H, multiplet);
7.11 (1H, doublet, J = 7.8 Hz);
6.25 (1H, doublet, J = 5.4 Hz);
6.20 (1H, doublet, J = 6.8 Hz);
5.24 (1H, doublet, J = 4.4 Hz);
5.20 (2H, singlet);
4.43 (1H, quintet, J = 7.3, 12.7 Hz);
3.61-3.93 (4H, multiplet).

EXAMPLE 20

2'-Cyano-2',3'-dideoxy-2',3'-didehydro-β-D-ribofuranosylcytosine

The procedure for producing the title compound of Example 12 was repeated, but using 300 mg of the title compound of Example 8, to obtain 60 mg of the title compound.
Nuclear Magnetic Resonance spectrum (270M Hz in hexadeuterated dimethyl sulphoxide) δppm:
7.79 (1H, doublet, J = 7.3 Hz);
7.55-7.56 (1H, multiplet);
7.36 (2H, doublet, J = 7.3 Hz);
7.07 (1H, doublet of doublets, J = 1.96, 3.90 Hz);
5.78 (1H, doublet, J = 7.3 Hz);
5.18-5.21 (1H, multiplet);
5.01-5.03 (1H, multiplet);
3.65-3.70 (2H, multiplet).

PREPARATIVE EXAMPLE 1

1-[2'-Cyano-2'-deoxy-5'-O-(4,4'-dimethoxytriphenylmethyl)-β-D-arabinofuranosyl]thymine

267 mg of the title compound of Example 3 was dissolved in 7 ml of anhydrous pyridine. 508 mg of 4,4'-dimethoxytriphenylmethyl chloride was added to the resulting solution, which was then stirred at room temperature for 1.5 hours in an argon gas stream. After completion of the reaction, the solvent was distilled off, and 100 ml of ethyl acetate was added to the residue. The mixture was washed three times with successive aliquots of 50 ml of water and then dried over anhydrous sodium sulphate. The solvent was evaporated and the residue purified on a silica gel column (ϕ1.8 x 8.5 cm) (eluted with ethanol/chloroform = 1-2 : 99-88) to obtain 574 mg of the title compound as a yellowish white foam.
Nuclear Magnetic Resonance spectrum ($CDCl_3$) δppm:

8.40 (1H, broad singlet);
7.50 (1H, doublet, J = 1.2 Hz);
4.77-7.26 (9H, multiplet);
6.90-6.80 (4H, multiplet);
6.27 (1H, doublet, J = 6.8 Hz);
4.74 (1H, doublet, J = 6.8 Hz);
3.93 (1H, multiplet);
3.79 (6H, singlet);
3.62 (1H, multiplet);
3.61 (1H, multiplet);
3.30 (2H, multiplet);
1.67 (1H, doublet, J = 1.2 Hz).

PREPARATIVE EXAMPLE 2

1-[2'-Cyano-2'-deoxy-5'-O-(4,4'-dimethoxytriphenylmethyl)-β-D-arabinofuranosyl]-$N^4$-acetylcytosine

The procedure of Preparative Example 1 was repeated, but using 194 mg of the title compound of Example 7. 326 mg of the title compound was obtained as a yellowish white foam.
Nuclear Magnetic Resonance spectrum ($CDCl_3$) δppm:

8.80 (1H, broad singlet);
8.19 (1H, doublet, J = 7.6 Hz);
7.41-7.14 and 6.89-6.76 (14H, multiplet);
6.30 (1H, doublet, J = 6.1 Hz);
4.79 (2H, multiplet);
4.08 (2H, multiplet);
3.79 (6H, singlet);
3.56 (2H, multiplet);
2.09 (3H, singlet).

PREPARATIVE EXAMPLE 3

1-[2'-Cyano-2',3'-dideoxy-2',3'-didehydro-5'-O-(4,4'-dimethoxytriphenylmethyl)-β-D-ribofuranosyl]thymine

200 mg of the compound prepared in Preparative Example 1 was dissolved in 3 ml of anhydrous dimethylformamide. 94 mg of thiocarbonyldiimidazole were added to the resulting solution which was the stirred at room temperature for 13 hours 40 minutes in an argon gas stream. After completion of the reaction, ethyl acetate was added to the reaction mixture, and the mixture was washed three times successively with water and dried over anhydrous sodium sulphate. The solvents were then evaporated, and the residue purified on a silica gel column (2ϕ x 6.5 cm) (eluted with hexane/ethyl acetate = 1 : 1 to 1 : 2) to obtain 162 mg of the title compound as a white caramel.
Nuclear Magnetic Resonance spectrum ($CDCl_3$) δppm:

8.40 (1H, broad singlet);
7.45 (1H, doublet, J = 1.1 Hz);
7.10 (1H, doublet, J = 1,8 Hz);
7.06 (1H, doublet of doublets, J = 1.8, 4.0 Hz);

5.10 (1H, double of double doublets, J = 2.6, 3.3, 4.0 Hz);
4.12 (3H, singlet);
3.61 (1H, doublet of doublets, J = 2.6, 11.0 Hz);
3.45 (1H, doublet of doublets, J = 3.3, 11.0 Hz);
1.90 (3H, doublet, J = 1.1 Hz).

PREPARATIVE EXAMPLE 4

1-[2'-Cyano-2',3'-dideoxy-2',3'-didehydro-5'-O-(4,4'-dimethoxytriphenylmethyl)-β-D-arabinofuranosyl]-$N^4$-acetylcytosine

The procedure of Preparative Example 3 was repeated, but using 326 mg of the compound prepared in Preparative Example 2. After purification, crystallisation from ether was performed, and 163 mg of the title compound was obtained as crystals.
Nuclear Magnetic Resonance spectrum (hexadeuterated dimethyl sulphoxide) δppm:
9.22 (1H, singlet);
8.16 (1H, doublet, J = 7.3 Hz);
7.35-7.22 (9H, multiplet);
6.95 (1H, doublet of doublets, J = 1.8, 4.0 Hz);
6.90-6.84 (5H, multiplet);
6.68 (1H, dt, J = 1.8);
5.08 (1H, double of double doublets, J = 2.6, 2.9, 4.0 Hz);
3.82 (6H, singlet);
3.71 (1H, doublet of doublets, J = 2.9, 11.7 Hz);
3.59 (1H, doublet of doublets, J = 2.6, 11.7 Hz);
2.24 (3H, singlet).

PREPARATIVE EXAMPLE 5

$N^4$-Benzyloxycarbonyl-2'-cyano-2'-phenoxythiocarbonyl-3',5'-O-TIPDS-β-D-arabinofuranosylcytosine

525 mg of the title compound of Example 17 was dissolved in pyridine, subsequent to which the moisture content was removed by azeotropic distillation. The residue was dissolved in 5 ml of methylene chloride and, to the resulting solution, were added 40 mg of dimethylaminopyridine, 0.17 ml of phenyl chlorothionoformate and 0.17 ml of triethylamine, in that order, with ice-cooling under a nitrogen gas stream. The mixture was then stirred for 4 hours with ice-cooling. Methylene chloride was added to the reaction mixture which was then washed successively with saturated aqueous sodium chloride, 0.1 N hydrochloric acid and saturated aqueous sodium chloride. The mixture was then dried over anhydrous magnesium sulphate, and the solvents were evaporated. The residue was purified by silica gel chromatography (eluted with methylene chloride/methanol = 99.5 : 0.5) to obtain 0.46 g of the title compound.
Nuclear Magnetic Resonance spectrum (270M Hz in hexadeuterated dimethyl sulphoxide) δppm:
11.02 (1H, broad singlet);
8.00 (1H, doublet, J = 7.8 Hz);
7.31-7.98 (10H, multiplet);
7.11 (1H, doublet, J = 7.8 Hz);
6.29 (1H, singlet);
5.75 (1H, broad singlet);
5.20 (2H, singlet);
3.98-4.17 (3H, multiplet);
1.00-1.12 (28H, multiplet).

FORMULATION EXAMPLE 1

Hard Capsules

Unit capsules of standard cap-and-body type hard gelatin capsules were prepared by charging each piece with: 100 mg of the powdery complex of Example 1; 150 mg of lactose; 50 mg of cellulose; and 6 mg of magnesium stearate. The resulting capsules were washed and dried to provide hard capsule preparations.

FORMULATION EXAMPLE 2

Tablets

100 mg of the complex of Example 1, 0.2 g of colloidal silicon dioxide, 5 mg of magnesium stearate, 275 mg of microcrystalline cellulose, 11 mg of starch and 98.8 mg of lactose were mixed and pelletised to form tablets.

If desired, a coating is applied to the tablets.

FORMULATION EXAMPLE 3

Injections

1.5 % by weight of the complex of Example 1 was stirred into 10 % by volume of propylene glycol. The mixture was then made up to a predetermined volume with distilled water for injection and sterilised to obtain a formulation for injection.

FORMULATION EXAMPLE 4

Suspension

100 mg of the micropowdery complex of Example 1, 100 mg of sodium carboxymethyl cellulose, 5 mg of sodium benzoate, 1.0 g of sorbitol solution (Pharmacopoeia of Japan) and 0.025 ml of vaniline were admixed in 5 ml of purified water and homogeneously suspended to obtain a suspension.

TEST EXAMPLE

1. Evaluation of Antitumour Activity in vitro

Antitumour activity was determined in vitro using a human cancer strain. An RPMI1640 solution containing 10% immobilised bovine foetal serum and 50 μg/ml kanamycin was employed as the culture medium. Cancer cells (1 x $10^4$ cells/ml) were inoculated in to 1 ml of the culture medium containing samples of compounds to be assayed at different concentrations, respectively, and cultured in a carbon dioxide gas incubator at 37°C for 72 hours.

Viability of the cancer cells was determined by the MTT method, in which the amount of living cells in the cultures containing samples of test compound is compared with blanks containing no test compound. Viable cells are measured based on the intensity of visible light using 3-(4,5-dimethylthiazol-2-yl)-2,5-diphenyltetrazolium bromide, the intensity of the resulting colour being dependent in proportion to the number of living cells. The intensity of antitumour activity was expressed as the $IC_{50}$ value [concentration (μg/ml) necessary for 50% inhibition of proliferation of the cells], obtained from a graph of % proliferation of the cancer cells of the group containing test sample (% relative to the blank group) against concentration (logarithmic) of the sample.

The results are summarised in Table 4.

Table 4

| Antitumour activity in vitro | | |
|---|---|---|
| Compound No. | $IC_{50}$ (μg/ml) | |
| | L1210 | K B |
| Example 9 | 0.21 | 15 |
| Example 12 | 3.1 | 7.6 |
| L1210: Mouse leukemia cell<br>KB: human oral epidermoid carcinoma | | |

## Claims

**1.** A compound having the general formula:

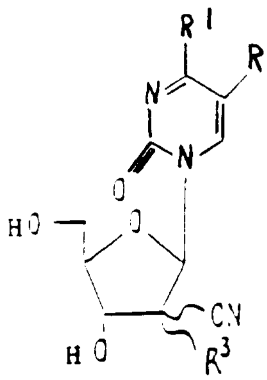

(I)

or the general formula:

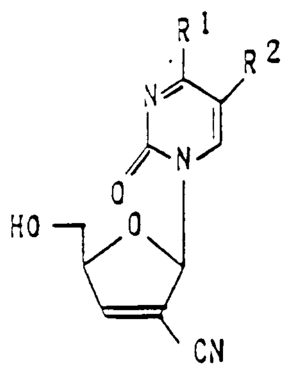

(II)

[wherein $R^1$ represents a hydroxyl group or an amino group optionally having a substituent selected from substituents (a) and (b) below;
$R^2$ represents a hydrogen atom or an alkyl group having 1 to 4 carbon atoms;
$R^3$ represents a hydrogen atom or a hydroxyl group; and
substituents (a)
aliphatic acyl groups having 1 to 4 carbon atoms, and aromatic acyl groups having 7 to 11 carbon atoms, the ring having an optional substituent thereon selected from benzoyl, α-naphthoyl and β-naphthoyl groups;
substituents (b)
alkoxycarbonyl groups wherein the alkyl part has from 1 to 4 carbon atoms, alkenyloxycarbonyl wherein

the alkenyl part has from 2 to 4 carbon atoms, aralkyloxycarbonyl groups having 8 to 12 carbon atoms, the ring having an optional substituent thereon selected from alkyl groups having from 1 to 4 carbon atoms, alkoxy groups having from 1 to 4 carbon atoms and aliphatic acyloxy groups having from 1 to 4 carbon atoms];
and pharmacologically acceptable non-toxic salts thereof.

**2.** A compound according to Claim 1, wherein $R^1$ represents a hydroxyl group or an amino group and optionally has a substituent selected from substituents (a') and (b') below;
<u>substituents (a')</u>
aliphatic acyl groups having 1 to 2 carbon atoms, and aromatic acyl groups having 7 carbon atoms, the ring having an optional substituent thereon as defined in claim 1;
<u>substituents (b')</u>
alkoxycarbonyl groups wherein the alkyl part has from 1 to 4 carbon atoms, alkenyloxycarbonyl groups wherein the alkenyl part has 3 carbon atoms, aralkyloxycarbonyl groups having 8 to 9 carbon atoms and which optionally have a substituent on the ring as defined in claim 1.

**3.** A compound according to Claim 2, wherein $R^1$ represents a hydroxyl group or an amino group and optionally has a substituent (a') as defined; $R^2$ represents a hydrogen atom or a methyl group.

**4.** A compound according to Claim 3, wherein $R^1$ represents a hydroxyl group or an amino group.

**5.** A compound according to Claim 1, selected from:
1-(2'-cyano-β-D-arabinofuranosyl)cytosine;
1-(2'-cyano-β-D-arabinofuranosyl)uracil;
1-(2'-cyano-β-D-arabinofuranosyl)thymine;
1-(2'-cyano-β-D-ribofuranosyl)cytosine;
1-(2'-cyano-β-D-ribofuranosyl)uracil;
1-(2'-cyano-β-D-ribofuranosyl)thymine;
1-(2'-cyano-β-D-2'-deoxyarabinofuranosyl)cytosine;
1-(2'-cyano-β-D-2'-deoxyarabinofuranosyl)uracil;
1-(2'-cyano-β-D-2'-deoxyarabinofuranosyl)thymine;
1-(2'-cyano-β-D-2'-deoxyribofuranosyl)cytosine;
1-(2'-cyano-β-D-2'-deoxyribofuranosyl)uracil;
1-(2'-cyano-β-D-2'-deoxyribofuranosyl)thymine;
1-(2'-cyano-β-D-2',3'-didehydro-2',3'-dideoxyribofuranosyl)cytosine;
1-(2'-cyano-β-D-2',3'-didehydro-2',3'-dideoxyribofuranosyl)uracil;
1-(2'-cyano-β-D-2',3'-didehydro-2',3'-dideoxyribofuranosyl)thymine;
and pharmacologically acceptable salts thereof.

**6.** An antitumour composition comprising an effective amount of a compound according to any of claims 1 to 5 together with a pharmaceutically acceptable carrier or excipient therefor.

**7.** Use of a compound according to any of claims 1 to 5 for the manufacture of a medicament for the treatment or prophylaxis of a tumour.

**8.** A compound of formula:

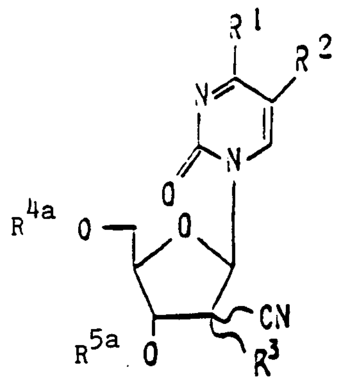

in which $R^1$, $R^2$ and $R^3$ are as defined in claim 1 and $R^{4a}$ and $R^{5a}$ together form a group $-R^6R^7Si-O-SiR^{6'}R^{7'}-$, wherein $R^6$, $R^{6'}$, $R^7$ and $R^{7'}$ are the same or different and each represents an alkyl group having from 1 to 4 carbon atoms.

**9.** A process for preparing a compound represented by the general formula:

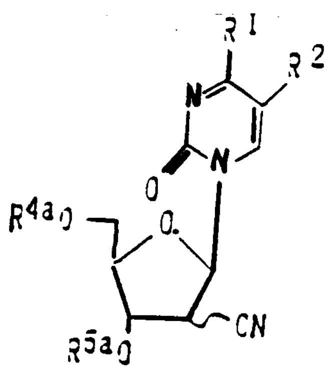

(wherein $R^1$ and $R^2$ are as defined in Claim 1; and $R^{4a}$ and $R^{5a}$ together represent a group of formula $-R^6R^7Si-O-SiR^{6'}R^{7'}-$ [wherein $R^6$, $R^{6'}$, $R^7$ and $R^{7'}$ are as defined in Claim 8]),
which process comprises reacting a compound represented by the general formula:

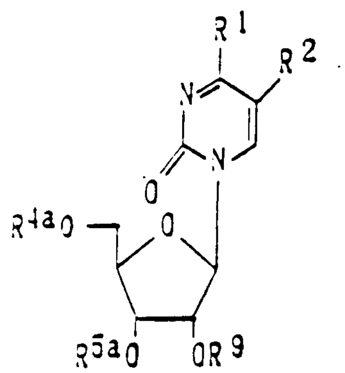

(wherein $R^1$, $R^2$, $R^{4a}$ and $R^{5a}$ are as defined above; and $R^9$ represents an alkoxythiocarbonyl group wherein the alkyl part has from 1 to 4 carbon atoms or an arylthiocarbonyl group wherein the aryl part has from 6 to 10 carbon atoms) with a reducing agent and a cyanolating agent.

**10.** A process for preparing a compound represented by the general formula:

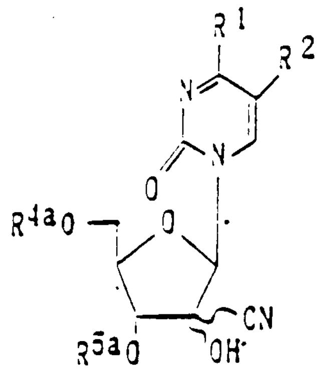

(wherein $R^1$ and $R^2$ are as defined in Claim 1, and $R^{4a}$ and $R^{5a}$ are as defined in Claim 8), which process comprises reacting a compound represented by the general formula:

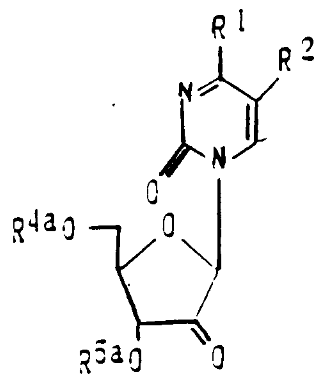

(wherein $R^1$, $R^2$, $R^{4a}$ and $R^{5a}$ ae as defined) with a cyanolating agent.

**11.** A process for preparing a compound represented by the general formula:

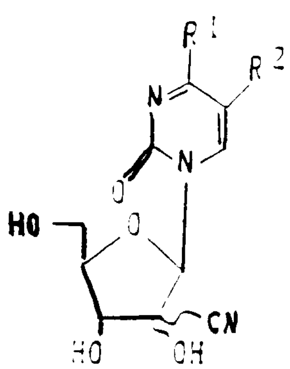

(wherein $R^1$ and $R^2$ are as defined in Claim 1),
which process comprises reacting a compound represented by the general formula:

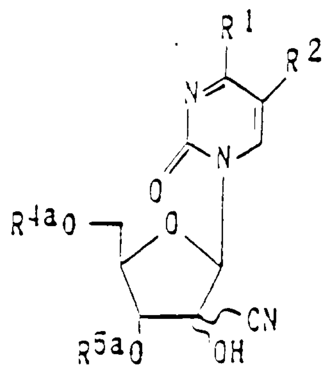

(wherein $R^1$ and $R^2$ are as defined; and $R^{4a}$ and $R^{5a}$ are as defined in Claim 8) with a deprotecting agent.

**12.** A process according to Claim 11 wherein the reactants and conditions are so selected as to produce a compound selected from:
1-(2'-cyano-β-D-arabinofuranosyl)cytosine,
1-(2'-cyano-β-D-arabinofuranosyl)uracil,
1-(2'-cyano-β-D-arabinofuranosyl)thymine,
1-(2'-cyano-β-D-ribofuranosyl)cytosine,
1-(2'-cyano-β-D-ribofuranosyl)uracil, and
1-(2'-cyano-β-D-ribofuranosyl)thymine.

**13.** A process for preparing a compound represented by the general formula:

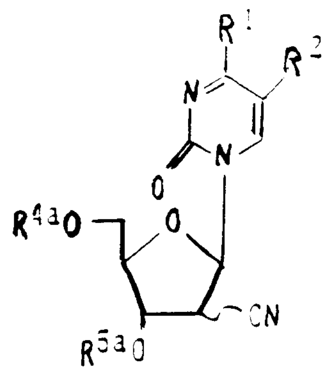

(wherein $R^1$ and $R^2$ are as defined in Claim 1, and $R^{4a}$ and $R^{5a}$ are as defined in Claim 8), which process comprises reacting a compound represented by the general formula:

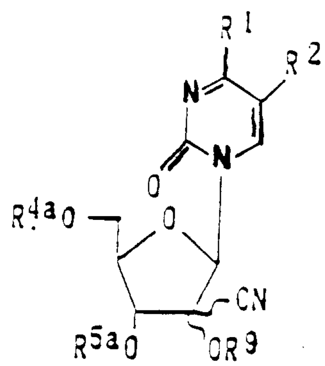

(wherein $R^1$, $R^2$, $R^{4a}$ and $R^{5a}$ are as defined; and $R^9$ represents an alkoxythiocarbonyl group wherein the alkyl part has from 1 to 4 carbon atoms or an arylthiocarbonyl group wherein the aryl part has from 6 to 10 carbon atoms) with a reducing agent.

**14.** A process for preparing a compound represented by the general formula:

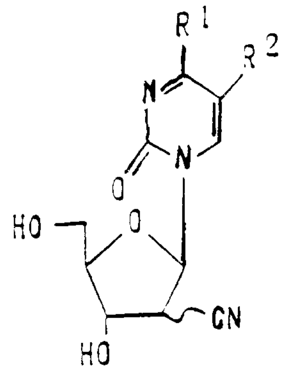

(wherein $R^1$ and $R^2$ are as defined in Claim 1), which process comprises reacting a compound represented by the general formula:

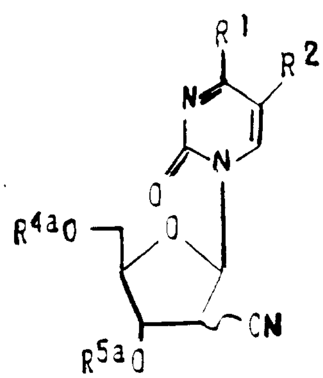

(wherein $R^1$ and $R^2$ are as defined in Claim 1, and $R^{4a}$ and $R^{5a}$ are as defined in Claim 8) with a deprotecting agent.

**15.** A process according to Claim 14 wherein the reactants and conditions are so selected as to produce a compound selected from:
1-(2'-cyano-β-D-2'-deoxyarabinofuranosyl)cytosine,
1-(2'-cyano-β-D-2'-deoxyarabinofuranosyl)uracil,
1-(2'-cyano-β-D-2'-deoxyarabinofuranosyl)thymine,
1-(2'-cyano-β-D-2'-deoxyribofuranosyl)cytosine,
1-(2'-cyano-β-D-2'-deoxyribofuranosyl)uracil, and
1-(2'-cyano-β-D-2'-deoxyribofuranosyl)thymine.

16. A process for preparing a compound represented by the general formula:

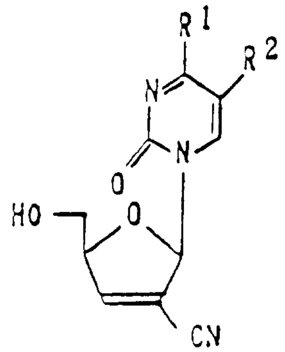

(wherein $R^1$ and $R^2$ are as defined in Claim 1), which process comprises reacting a compound represented by the general formula:

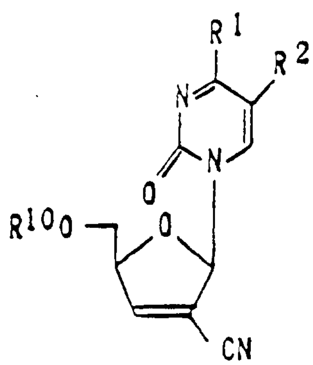

(wherein $R^1$ and $R^2$ are as defined, and $R^{10}$ represents a protective group) with a deprotecting agent.

17. A process according to Claim 16 wherein the reactants and conditions are so selected as to produce a compound selected from:
1-(2'-cyano-β-D-2',3'-didehydro-2',3'-dideoxyribofuranosyl)cytosine,
1-(2'-cyano-β-D-2',3'-didehydro-2',3'-dideoxyribofuranosyl)uracil and
1-(2'-cyano-β-D-2',3'-didehydro-2',3'-dideoxyribofuranosyl)thymine.

18. A process for preparing a compound represented by the general formula:

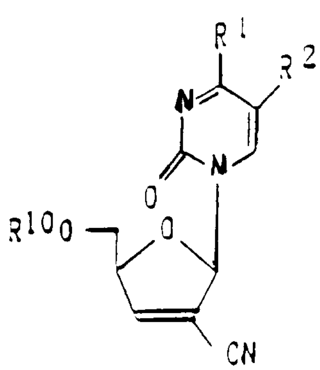

(wherein $R^1$ and $R^2$ are as defined in Claim 1, and $R^{10}$ is as defined in Claim 16), which process comprises reacting a compound represented by the general formula:

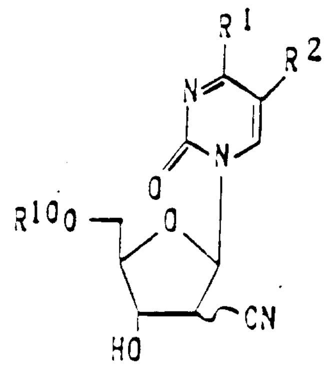

(wherein $R^1$, $R^2$ and $R^{10}$ are as defined) with an alkoxythiocarbonyl halide having a $C_1$ - $C_4$ alkyl moiety, an arylthiocarbonyl halide having a $C_6$ - $C_{10}$ aryl moiety, or thiocarbonyldiimidazole.

**19.** A compound according to any of claims 1 to 5 for use in therapy.

**Patentansprüche**

**1.** Verbindung der allgemeinen Formel

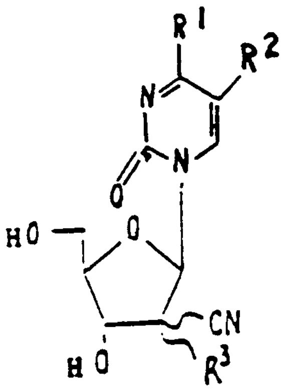

(I)

oder der allgemeinen Formel

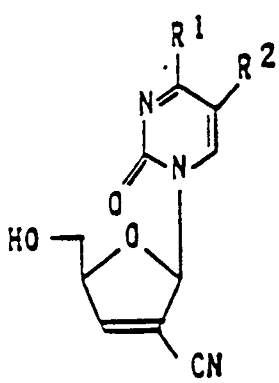

(II)

worin $R^1$ eine Hydroxylgruppe oder eine Aminogruppe, die gegebenenfalls einen unter den nachstehenden Substituenten (a) und (b) ausgewählten Substituenten aufweist, bedeutet,
$R^2$ ein Wasserstoffatom oder eine Alkylgruppe mit 1 bis 4 Kohlenstoffatomen bedeutet,
$R^3$ ein Wasserstoffatom oder eine Hydroxylgruppe bedeutet, und die Substituenten folgende Bedeutung haben:
<u>Substituenten (a)</u>

aliphatische Acylgruppen mit 1 bis 4 Kohlenstoffatomen und aromatische Acylgruppen mit 7 bis 11 Kohlenstoffatomen, in denen der Ring gegebenenfalls einen Substituenten aufweist, der unter Benzoyl-, α-Naphthoyl- und β-Naphthoyl-Gruppen ausgewählt ist,
Substituenten (b)
Alkoxycarbonylgruppen, deren Alkylteil 1 bis 4 Kohlenstoffatome aufweist, Alkenyloxycarbonylgruppen, deren Alkenylteil 2 bis 4 Kohlenstoffatome enthält, Aralkyloxycarbonylgruppen mit 8 bis 12 Kohlenstoffatomen, deren Ring gegebenenfalls einen Substituenten aufweist, der unter Alkylgruppen mit 1 bis 4 Kohlenstoffatomen, Alkoxygruppen mit 1 bis 4 Kohlenstoffatomen und aliphatischen Acyloxygruppen mit 1 bis 4 Kohlenstoffatomen ausgewählt ist,
und pharmakologisch geeignete nicht-toxische Salze dieser Verbindung.

**2.** Verbindung nach Anspruch 1, worin $R^1$ eine Hydroxylgruppe oder eine Aminogruppe bedeutet und gegebenenfalls einen unter den nachstehend definierten Substituenten (a') und (b') ausgewählten Substituenten aufweist:
Substituenten (a')
aliphatische Acylgruppen mit 1 bis 2 Kohlenstoffatomen und aromatische Acylgruppen mit 7 Kohlenstoffatomen, wobei der Ring gegebenenfalls einen wie in Anspruch 1 definierten Substituenten aufweist,
Substituenten (b')
Alkoxycarbonylgruppen, deren Alkylteil 1 bis 4 Kohlenstoffatome aufweist, Alkenyloxycarbonylgruppen, deren Alkenylteil 3 Kohlenstoffatome enthält, Aralkyloxycarbonylgruppen mit 8 bis 9 Kohlenstoffatomen, die gegebenenfalls am Ring einen wie in Anspruch 1 definierten Substituenten aufweisen.

**3.** Verbindung nach Anspruch 2, worin $R^1$ eine Hydroxylgruppe oder eine Aminogruppe bedeutet und gegebenenfalls einen wie bereits definierten Substituenten (a') aufweist, $R^2$ ein Wasserstoffatom oder eine Methylgruppe bedeutet.

**4.** Verbindung nach Anspruch 3, worin $R^1$ eine Hydroxylgruppe oder eine Aminogruppe bedeutet.

**5.** Verbindung nach Anspruch 1, die ausgewählt ist unter:
1-(2'-Cyan-β-D-arabinofuranosyl)cytosin,
1-(2'-Cyan-β-D-arabinofuranosyl)uracil,
1-(2'-Cyan-β-D-arabinofuranosyl)thymin,
1-(2'-Cyan-β-D-ribofuranosyl)cytosin,
1-(2'-Cyan-β-D-ribofuranosyl)uracil,
1-(2'-Cyan-β-D-ribofuranosyl)thymin,
1-(2'-Cyan-β-D-2'-desoxyarabinofuranosyl)cytosin,
1-(2'-Cyan-β-D-2'-desoxyarabinofuranosyl)uracil,
1-(2'-Cyan-β-D-2'-desoxyarabinofuranosyl)thymin,
1-(2'-Cyan-β-D-2'-desoxyribofuranosyl)cytosin,
1-(2'-Cyan-β-D-2'-desoxyribofuranosyl)uracil,
1-(2'-Cyan-β-D-2'-desoxyribofuranosyl)thymin,
1-(2'-Cyan-β-D-2',3'-didehydro-2',3'-didesoxy ribofuranosyl)cytosin,
1-(2'-Cyan-β-D-2',3'-didehydro-2',3'-didesoxy ribofuranosyl)uracil,
1-(2'-Cyan-β-D-2',3'-didehydro-2',3'-didesoxy ribofuranosyl)thymin
und deren pharmakologisch geeignete Salze.

**6.** Antitumor-Zusammensetzung, enthaltend eine wirksame Menge einer Verbindung nach einem der Ansprüche 1 bis 5 zusammen mit einem pharmazeutisch geeigneten Trägermaterial oder Exzipienten dafür.

**7.** Verwendung einer Verbindung nach einem der Ansprüche 1 bis 5 zur Herstellung eines Arzneimittels zur Behandlung oder Prophylaxe eines Tumors.

**8.** Verbindung der Formel:

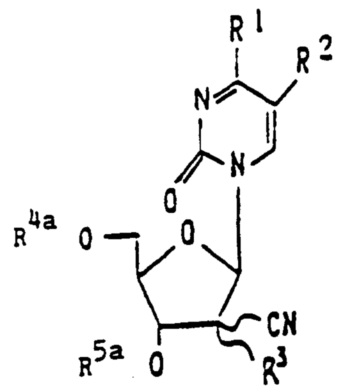

worin $R^1$, $R^2$ und $R^3$ wie in Anspruch 1 definiert sind und $R^{4a}$ und $R^{5a}$ zusammen eine Gruppe $-R^6R^7Si-O-SiR^{6'}R^{7'}-$ bilden, in der $R^6$, $R^{6'}$, $R^7$ und $R^{7'}$ gleich oder verschieden sind und jeweils eine Alkylgruppe mit 1 bis 4 Kohlenstoffatomen bedeuten.

**9.** Verfahren zur Herstellung einer Verbindung der allgemeinen Formel:

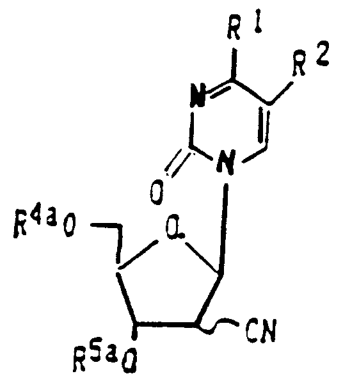

(worin $R^1$ und $R^2$ wie in Anspruch 1 definiert sind und $R^{4a}$ und $R^{5a}$ zusammen eine Gruppe der Formel $-R^6R^7Si-O-SiR^{6'}R^{7'}-$ bilden [in der $R^6$, $R^{6'}$, $R^7$ und $R^{7'}$ wie in Anspruch 8 definiert sind]),
bei dem eine Verbindung der allgemeinen Formel:

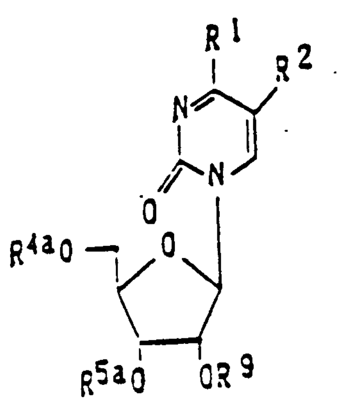

(worin $R^1$, $R^2$, $R^{4a}$, $R^{5a}$ wie oben definiert sind und $R^9$ eine Alkoxythiocarbonylgruppe bedeutet, deren Alkylteil 1 bis 4 Kohlenstoffatome hat oder eine Arylthiocarbonylgruppe bedeutet, deren Arylteil 6 bis 10 Kohlenstoffatome hat) mit einem Reduktionsmittel und einem Cyanierungsmittel behandelt wird.

**10.** Verfahren zur Herstellung einer Verbindung der allgemeinen Formel:

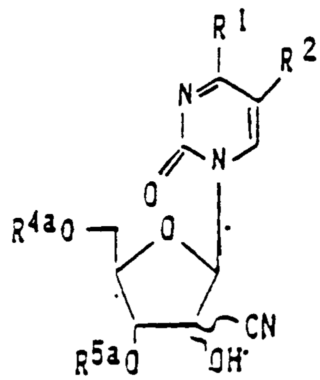

(worin $R^1$ und $R^2$ wie in Anspruch 1 definiert sind, und $R^{4a}$ und $R^{5a}$ wie in Anspruch 8 definiert sind), bei dem eine Verbindung der allgemeinen Formel:

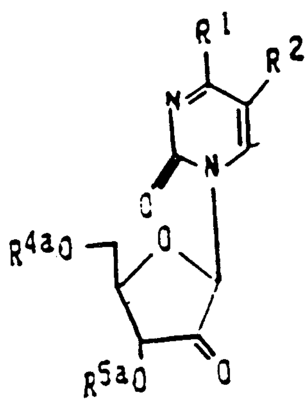

(in der $R^1$, $R^2$, $R^{4a}$, $R^{5a}$ wie oben definiert sind) mit einem Cyanierungsmittel behandelt wird.

**11.** Verfahren zur Herstellung einer Verbindung der allgemeinen Formel:

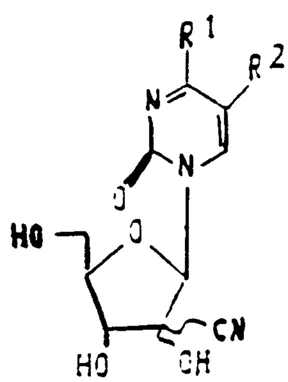

(worin $R^1$ und $R^2$ wie in Anspruch 1 definiert sind), bei dem eine Verbindung der allgemeinen Formel:

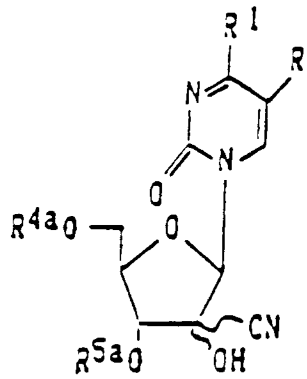

(worin $R^1$ und $R^2$ wie vorher definiert sind und $R^{4a}$ und $R^{5a}$ wie in Anspruch 8 definiert sind) mit einem Mittel zum Entfernen der Schutzgruppe umgesetzt wird.

**12.** Verfahren nach Anspruch 11, bei dem die Reaktanten und Bedingungen so gewählt werden, daß eine Verbindung hergestellt wird, die ausgewählt ist unter
1-(2'-Cyan-β-D-arabinofuranosyl)cytosin,
1-(2'-Cyan-β-D-arabinofuranosyl)uracil,
1-(2'-Cyan-β-D-arabinofuranosyl)thymin,
1-(2'-Cyan-β-D-ribofuranosyl)cytosin,
1-(2'-Cyan-β-D-ribofuranosyl)uracil und
1-(2'-Cyan-β-D-ribofuranosyl)thymin.

**13.** Verfahren zur Herstellung einer Verbindung der allgemeinen Formel:

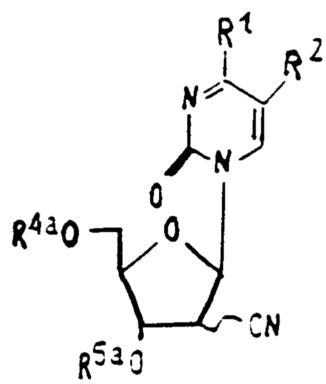

(worin $R^1$ und $R^2$ wie in Anspruch 1 definiert sind und $R^{4a}$ und $R^{5a}$ wie in Anspruch 8 definiert sind), bei dem eine Verbindung der allgemeinen Formel:

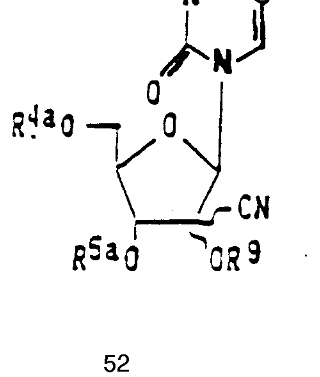

(worin $R^1$, $R^2$, $R^{4a}$ und $R^{5a}$ wie bereits definiert sind und $R^9$ eine Alkoxythiocarbonylgruppe, deren Alkylteil 1 bis 4 Kohlenstoffatome enthält, oder eine Arylthiocarbonylgruppe bedeutet, deren Arylteil 6 bis 10 Kohlenstoffatome enthält) mit einem Reduktionsmittel umgesetzt wird.

**14.** Verfahren zur Herstellung einer Verbindung der allgemeinen Formel:

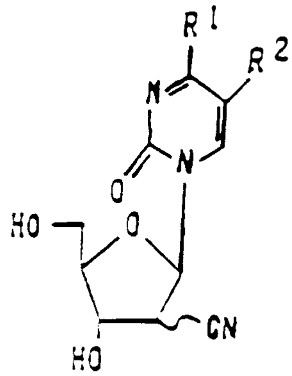

(worin $R^1$ und $R^2$ wie in Anspruch 1 definiert sind), bei dem eine Verbindung der allgemeinen Formel:

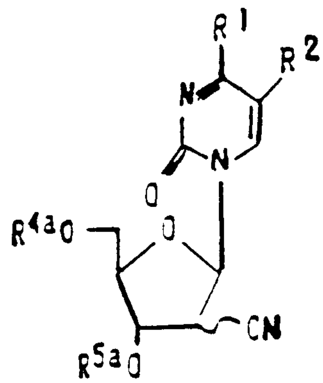

(worin $R^1$ und $R^2$ wie in Anspruch 1 definiert sind und $R^{4a}$ und $R^{5a}$ wie in Anspruch 8 definiert sind) mit einem Mittel zum Entfernen der Schutzgruppe umgesetzt wird.

**15.** Verfahren nach Anspruch 14, bei dem die Reaktanten und Bedingungen so gewählt werden, daß eine Verbindung hergestellt wird, die ausgewählt ist unter:
1-(2'-Cyan-β-D-2'-desoxyarabinofuranosyl)cytosin,
1-(2'-Cyan-β-D-2'-desoxyarabinofuranosyl)uracil,
1-(2'-Cyan-β-D-2'-desoxyarabinofuranosyl)thymin,
1-(2'-Cyan-β-D-2'-desoxyribofuranosyl)cytosin,
1-(2'-Cyan-β-D-2'-desoxyribofurarosyl)uracil und
1-(2'-Cyan-β-D-2'-desoxyribofuranosyl)thymin.

**16.** Verfahren zur Herstellung einer Verbindung der allgemeinen Formel:

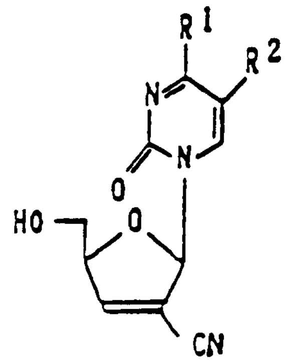

(worin $R^1$ und $R^2$ wie in Anspruch 1 definiert sind), gemäß dem eine Verbindung der allgemeinen Formel:

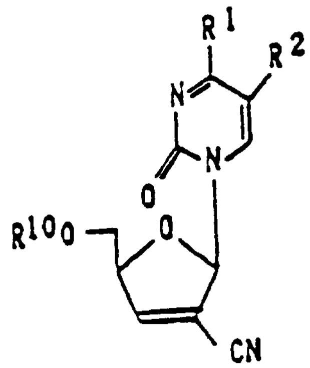

(worin $R^1$ und $R^2$ wie bereits definiert sind und $R^{10}$ eine Schutzgruppe bedeutet) mit einem Mittel zum Entfernen der Schutzgruppe umgesetzt wird.

**17.** Verfahren nach Anspruch 16, bei dem die Reaktanten und Bedingungen so gewählt werden, daß eine Verbindung hergestellt wird, die ausgewählt ist unter:

1-(2'-Cyan-β-D-2',3'-didehydro-2',3'-didesoxyribofuranosyl)cytosin,
1-(2'-Cyan-β-D-2',3'-didehydro-2',3'-didesoxyribofuranosyl)uracil und
1-(2'-Cyan-β-D-2',3'-didehydro-2',3'-didesoxyribofuranosyl)thymin.

**18.** Verfahren zur Herstellung einer Verbindung der allgemeinen Formel:

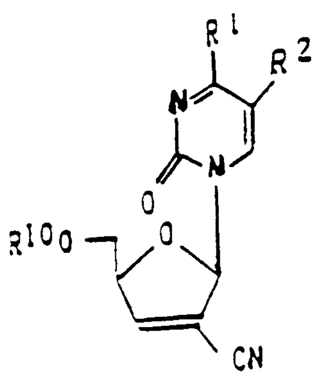

(worin $R^1$ und $R^2$ wie in Anspruch 1 definiert sind und $R^{10}$ wie in Anspruch 16 definiert ist),

gemäß dem eine Verbindung der allgemeinen Formel:

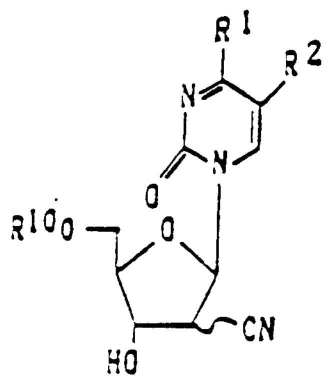

(worin $R^1$, $R^2$ und $R^{10}$ wie bereits definiert sind) mit einem Alkoxythiocarbonylhalogenid, das eine $C_1$-$C_4$-Alkyleinheit enthält, einem Arylthiocarbonylhalogenid, das eine $C_6$-$C_{10}$-Aryleinheit enthält, oder mit Thiocarbonyldiimidazol umgesetzt wird.

**19.** Verbindung nach einem der Ansprüche 1 bis 5 zur therapeutischen Verwendung.

**Revendications**

**1.** Composé répondant à la formule générale :

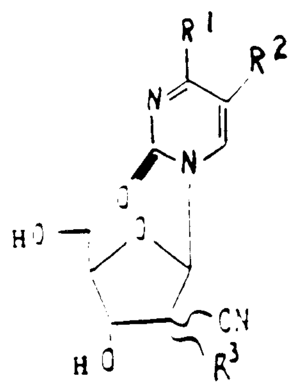

(I)

ou à la formule générale :

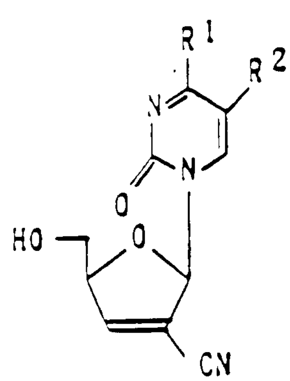

(II)

[où $R^1$ représente un groupe hydroxy ou un groupe amino ayant facultativement un substituant choisi parmi les substituants (a) et (b) ci-après ;

$R^2$ représente un atome d'hydrogène ou un groupe alkyle ayant 1 à 4 atomes de carbone ;
$R^3$ représente un atome d'hydrogène ou un groupe hydroxy ; et
substituants (a)
groupes acyle aliphatiques ayant 1 à 4 atomes de carbone et groupes acyle aromatiques ayant 7 à 11 atomes de carbone, le cycle portant un substituant facultatif choisi parmi les groupes benzoyle, α-naphtoyle et β-naphtoyle ;
substituants (b)
groupes alcoxycarbonyle dont la partie alkyle a de 1 à 4 atomes de carbone, groupes alcényloxycarbonyle dont la partie alcényle a de 2 à 4 atomes de carbone, groupes aralkyloxycarbonyle ayant 8 à 12 atomes de carbone, le cycle portant un substituant facultatif choisi parmi les groupes alkyle ayant de 1 à 4 atomes de carbone, les groupes alcoxy ayant de 1 à 4 atomes de carbone et les groupes acyloxy aliphatiques ayant de 1 à 4 atomes de carbone] ;
et ses sels non toxiques pharmacologiquement acceptables.

**2.** Composé selon la revendication 1, où $R^1$ représente un groupe hydroxy ou un groupe amino et a facultativement un substituant choisi parmi les substituants (a') et (b') ci-après
substituants (a')
groupes acyle aliphatiques ayant 1 ou 2 atomes de carbone et groupes acyle aromatiques ayant 7 atomes de carbone, le cycle portant un substituant facultatif tel que défini dans la revendication 1 ;
substituants (b')
groupes alcoxycarbonyle dont la partie alkyle a de 1 à 4 atomes de carbone, groupes alcényloxycarbonyle dont la partie alcényle a 3 atomes de carbone, groupes aralkyloxycarbonyle ayant 8 ou 9 atomes de carbone et qui portent facultativement un substituant sur le cycle tel que défini dans la revendication 1.

**3.** Composé selon la revendication 2, où $R^1$ représente un groupe hydroxy ou un groupe amino et a facultativement un substituant (a') tel que défini ; et $R^2$ représente un atome d'hydrogène ou un groupe méthyle.

**4.** Composé selon la revendication 3, où $R^1$ représente un groupe hydroxy ou un groupe amino.

**5.** Composé selon la revendication 1 choisi parmi :
la 1-(2'-cyano-β-D-arabinofurannosyl)cytosine ;
le 1-(2'-cyano-β-D-arabinofurannosyl)uracile ;
la 1-(2'-cyano-β-D-arabinofurannosyl)thymine ;
la 1-(2'-cyano-β-D-ribofurannosyl)cytosine ;
le 1-(2'-cyano-β-D-ribofurannosyl)uracile ;
la 1-(2'-cyano-β-D-ribofurannosyl)thymine ;
la 1-(2'-cyano-β-D-2'-désoxyarabinofurannosyl)cytosine ;
le 1-(2'-cyano-β-D-2'-désoxyarabinofurannosyl)uracile ;
la 1-(2'-cyano-β-D-2'-désoxyarabinofurannosyl)thymine ;
la 1-(2'-cyano-β-D-2'-désoxyribofurannosyl)cytosine ;
le 1-(2'-cyano-β-D-2'-désoxyribofurannosyl)uracile ;
la 1-(2'-cyano-β-D-2'-désoxyribofurannosyl)thymine ;
la 1-(2'-cyano-β-D-2',3'-didéhydro-2',3'-didésoxyribofurannosyl) cityosine ;
le 1-(2'-cyano-β-D-2',3'-didéhydro-2',3'-didésoxyribofurannosyl) uracile ;
la 1-(2'-cyano-β-D-2',3'-didéhydro-2',3'-didésoxyribofurannosyl) thymine ;
et leurs sels pharmacologiquement acceptables.

**6.** Composition antitumorale comprenant une quantité efficace d'un composé selon l'une quelconque des revendications 1 à 5 avec un véhicule ou excipient pharmaceutiquement acceptables.

**7.** Utilisation d'un composé selon l'une quelconque des revendications 1 à 5 pour la préparation d'un médicament pour le traitement ou pour la prophylaxie d'une tumeur.

**8.** Composé de formule :

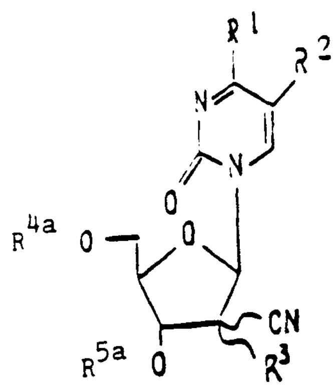

dans laquelle $R^1$, $R^2$ et $R^3$ sont tels que définis dans la revendication 1 et $R^{4a}$ et $R^{5a}$ forment ensemble un groupe $-R^6R^7Si-O-SiR^{6'}R^{7'}-$, où $R^6$, $R^{6'}$, $R^7$ et $R^{7'}$ sont identiques ou différents et représentent chacun un groupe alkyle ayant de 1 à 4 atomes de carbone.

**9.** Procédé pour préparer un composé représenté par la formule générale :

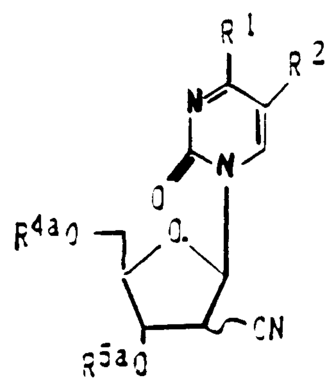

(dans laquelle $R^1$ et $R^2$ sont tels que définis dans la revendication 1 ; et $R^{4a}$ et $R^{5a}$ représentent ensemble un groupe de formule $-R^6R^7Si-O-SiR^{6'}R^{7'}-$ [dans laquelle $R^6$, $R^{6'}$, $R^7$ et $R^{7'}$ sont tels que définis dans la revendication 8]),
lequel procédé comprend la réaction d'un composé représenté par la formule générale :

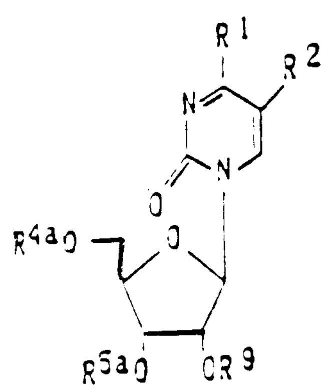

(dans laquelle $R^1$, $R^2$, $R^{4a}$ et $R^{5a}$ sont tels que définis ci-dessus ; et $R^9$ représente un groupe alcoxythiocarbonyle dont la partie alkyle a de 1 à 4 atomes de carbone ou un groupe arylthiocarbonyle dont la partie aryle a de 6 à 10 atomes de carbone) avec un agent réducteur et un agent de

cyanolation.

**10.** Procédé pour préparer un composé représenté par la formule générale :

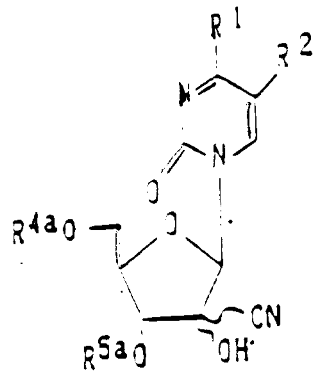

(dans laquelle $R^1$ et $R^2$ sont tels que définis dans la revendication 1 et $R^{4a}$ et $R^{5a}$ sont tels que définis dans la revendication 8),
lequel procédé comprend la réaction d'un composé représenté par la formule générale :

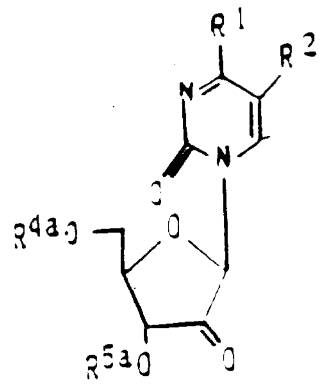

(dans laquelle $R^1$, $R^2$, $R^{4a}$ et $R^{5a}$ sont tels que définis) avec un agent de cyanolation.

**11.** Procédé pour préparer un composé représenté par la formule générale :

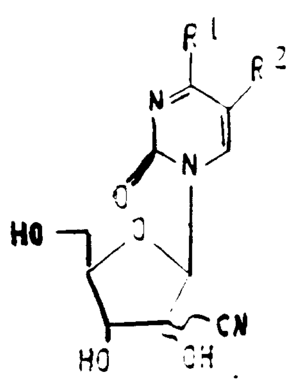

(dans laquelle $R^1$ et $R^2$ sont tels que définis dans la revendication 1),
lequel procédé comprend la réaction d'un composé représenté par la formule générale :

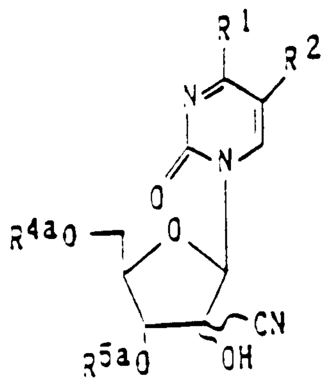

(dans laquelle $R^1$ et $R^2$ sont tels que définis ; et $R^{4a}$ et $R^{5a}$ sont tels que définis dans la revendication 8) avec un agent de déprotection.

**12.** Procédé selon la revendication 11, dans lequel les composés réagissants et les conditions sont choisis pour produire un composé choisi parmi :
la 1-(2'-cyano-β-D-arabinofurannosyl)cytosine,
le 1-(2'-cyano-β-D-arabinofurannosyl)uracile,
la 1-(2'-cyano-β-D-arabinofurannosyl)thymine,
la 1-(2'-cyano-β-D-ribofurannosyl)cytosine,
le 1-(2'-cyano-β-D-ribofurannosyl)uracile et
la 1-(2'-cyano-β-D-ribofurannosyl)thymine.

**13.** Procédé pour préparer un composé représenté par la formule générale :

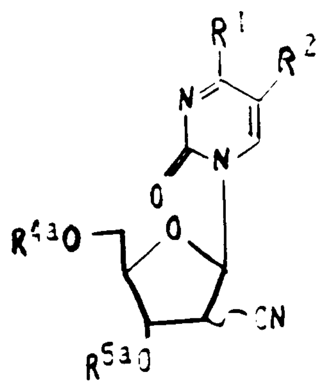

(dans laquelle $R^1$ et $R^2$ sont tels que définis dans la revendication 1 et $R^{4a}$ et $R^{5a}$ sont tels que définis dans la revendication 8),
lequel procédé comprend la réaction d'un composé représenté par la formule générale :

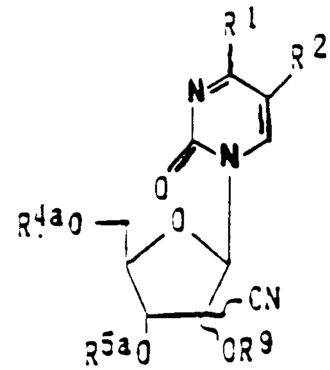

(dans laquelle $R^1$ et $R^2$, $R^{4a}$ et $R^{5a}$ sont tels que définis ; et $R^9$ représente un groupe alcoxythiocarbonyle dont la partie alkyle a de 1 à 4 atomes de carbone ou un groupe arylthiocarbonyle dont la partie aryle a de 6 à 10 atomes de carbone) avec un agent réducteur.

**14.** Procédé pour préparer un composé représenté par la formule générale :

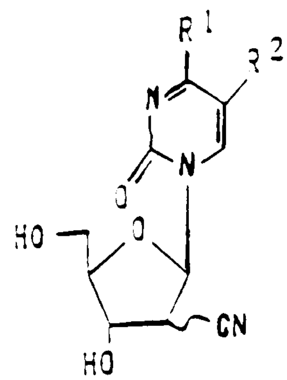

(dans laquelle $R^1$ et $R^2$ sont tels que définis dans la revendication 1),
lequel procédé comprend la réaction d'un composé représenté par la formule générale :

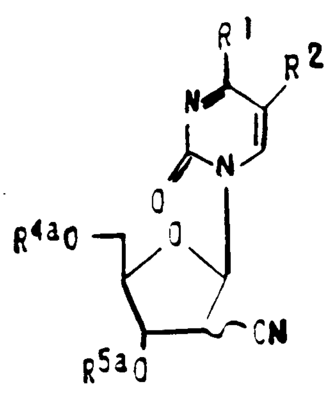

(dans laquelle $R^1$ et $R^2$ sont tels que définis dans la revendication 1 et $R^{4a}$ et $R^{5a}$ sont tels que définis dans la revendication 8) avec un agent de déprotection.

**15.** Procédé selon la revendication 14, dans lequel les composés réagissants et les conditions sont choisis pour produire un composé choisi parmi :
la 1-(2'-cyano-β-D-2'-désoxyarabinofurannosyl)cytosine,
le 1-(2'-cyano-β-D-2'-désoxyarabinofurannosyl)uracile,

la 1-(2'-cyano-β-D-2'-désoxyarabinofurannosyl)thymine,
la 1-(2'-cyano-β-D-2'-désoxyribofurannosyl)cytosine,
le 1-(2'-cyano-β-D-2'-désoxyribofurannosyl)uracile et
la 1-(2'-cyano-β-D-2'-désoxyribofurannosyl)thymine.

**16.** Procédé pour préparer un composé représenté par la formule générale :

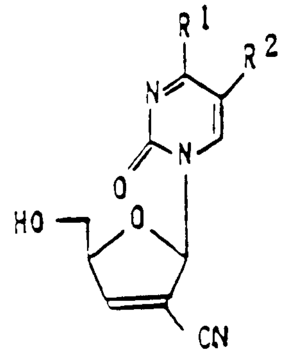

(dans laquelle $R^1$ et $R^2$ sont tels que définis dans la revendication 1),
lequel procédé comprend la réaction d'un composé représenté par la formule générale :

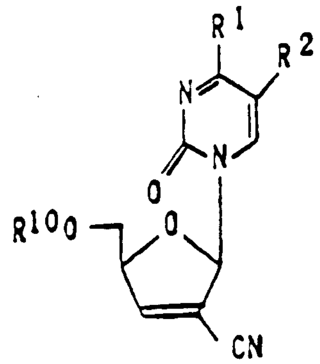

(dans laquelle $R^1$ et $R^2$ sont tels que définis et $R^{10}$ représente un groupe protecteur) avec un agent de déprotection.

**17.** Procédé selon la revendication 16, dans lequel les composés réagissants et les conditions sont choisis pour produire un composé choisi parmi :
la 1-(2'-cyano-β-D-2',3'-didéhydro-2',3'-didésoxyribofurannosyl) cytosine,
le 1-(2'-cyano-β-D-2',3'-didéhydro-2',3'-didésoxyribofurannosyl) uracile et
la 1-(2'-cyano-β-D-2',3'-didéhydro-2',3'-didésoxyribofurannosyl) thymine.

**18.** Procédé pour préparer un composé représenté par la formule générale :

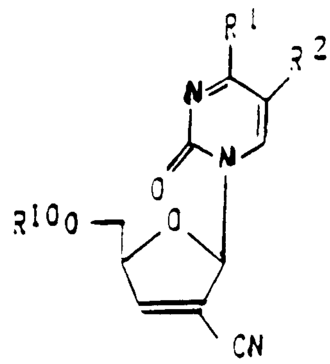

(dans laquelle $R^1$ et $R^2$ sont tels que définis dans la revendication 1 et $R^{10}$ est tel que défini dans la revendication 16),
lequel procédé comprend la réaction d'un composé représenté par la formule générale :

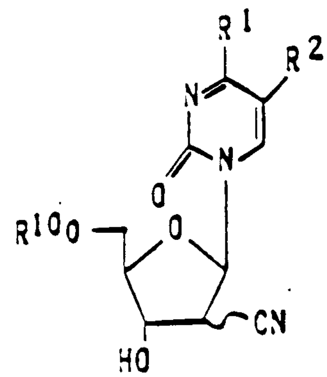

(dans laquelle $R^1$, $R^2$ et $R^{10}$ sont tels que définis) avec un halogénure d'alcoxythiocarbonyle ayant un fragment alkyle en $C_1$-$C_4$, un halogénure d'arylthiocarbonyle ayant un fragment aryle en $C_6$-$C_{10}$ ou le thiocarbonyldiimidazole.

**19.** Composé selon l'une quelconque des revendications 1 à 5 pour l'utilisation en thérapeutique.